# EUROPÄISCHE PATENTSCHRIFT

(11) **EP 3 319 951 B1**
(45) Veröffentlichungstag und Bekanntmachung des Hinweises auf die Patenterteilung: **08.09.2021**
(21) Anmeldenummer: 16736463.7
(22) Anmeldetag: 08.07.2016
(51) Int. Cl.: C07D 307/92, C07C 45/50, C07C 47/21, C07C 45/49, C07C 45/69

(54) **VERFAHREN ZUR HYDROFORMYLIERUNG VON 2-SUBSTITUIERTEN BUTADIENEN UND ZUR HERSTELLUNG VON FOLGEPRODUKTEN DAVON, SPEZIELL VON AMBROX**
METHOD FOR THE HYDROFORMYLATION OF 2-SUBSTITUTED BUTADIENES AND FOR THE PREPARATION OF PRODUCTS DERIVED FROM SAME, PARTICULARLY FROM AMBROX
PROCEDE D'HYDROFORMYLATION DE 2 BUTADIENES SUBSTITUES ET DESTINE A LA FABRICATIONS DE PRODUITS SECONDES, EN PARTICULIER D'AMBROXOL

(30) Priorität: 10.07.2015 EP 15176280
(43) Veröffentlichungstag der Anmeldung: 16.05.2018
(73) Patentinhaber: BASF SE, 67056 Ludwigshafen am Rhein (DE); Ruprecht-Karls-Universität Heidelberg, 69117 Heidelberg (DE)
(72) Erfinder: STRAUTMANN, Julia, 91054 Erlangen (DE); RUEDENAUER, Stefan, 69469 Weinheim (DE); REIN, Christian, 68542 Heddesheim (DE); WEINGARTEN, Melanie, 23909 Ratzeburg (DE); PACIELLO, Rocco, 67098 Bad Dürkheim (DE); SIEGEL, Wolfgang, 67117 Limburgerhof (DE); BREUER, Michael, 64285 Darmstadt (DE); HOFMANN, Peter, 69115 Heidelberg (DE); SCHMIDT, Sebastian, 40764 Langenfeld (DE)
(74) Vertreter: Reitstötter Kinzebach
(86) Internationale Anmeldenummer: PCT/EP2016/066225
(87) Internationale Veröffentlichungsnummer: WO 2017/009205

(56) Entgegenhaltungen:
- WO-A1-99/50214
- WO-A2-2010/033976
- US-A1- 2006 224 000
- US-A1- 2013 273 619
- C. M. FOCA; H. J. V. BARROS; E. N. DOS SANTOS; E. V. GUSEVSKAYA; J. C. BAYLON: "Hydroformylation of myrcene: metal and ligand effects in the hydroformylation of conugated dienes.", NEW J. CHEM., Bd. 27, 2003, Seiten 533-539, XP002752385, in der Anmeldung erwähnt

## Beschreibung

### HINTERGRUND DER ERFINDUNG

Die vorliegende Erfindung betrifft ein Verfahren zur regioselektiven Hydroformylierung mehrfach ungesättigter acyclischer Kohlenwasserstoffe, wobei es sich um 1,3-Butadiene handelt, die in der 2-Position einen gesättigten oder ein- oder mehrfach ungesättigten acyclischen Kohlenwasserstoffrest tragen. Die vorliegende Erfindung betrifft weiterhin die Herstellung von Folgeprodukten dieser Hydroformylierungsprodukte und speziell von Ambrox.

### STAND DER TECHNIK

Die Hydroformylierung von Butadien-Verbindungen der allgemeinen Formel A 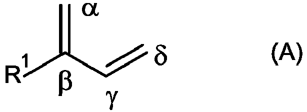 die in der beta-Position (oder 2-Position) einen Alkyl-, oder einen ein- oder mehrfach ungesättigten Alkenylrest tragen, beispielsweise Isopren oder Myrcen, führt unter Verwendung der bekannten Rhodium-Katalysatoren zu einem Isomerengemisch. Im Folgenden wird für eine bessere Verständlichkeit bei der Bezeichnung der Substituentenpositionen sowohl der Edukte als auch der Hydroformylierungsprodukte die oben abgebildete Bezeichnung in der Butadien-Ausgangsverbindung (A) beibehalten.

Das Hauptprodukt entsteht in den meisten literaturbekannten Synthesen durch Hydroformylierung in der alpha-Position, bei gleichzeitiger Isomerisierung der Doppelbindung. Im Fall von Isopren (B1) ist das Hauptprodukt daher meist das 3-Methyl-pentenal (B2) und im Fall von β-Myrcen (= 7-Methyl-3-methylen-1,6-octadien) (C1) das 3-Ethyliden-7-methyl-oct-6-enal (C2):

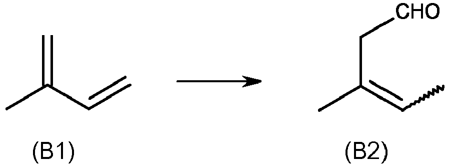

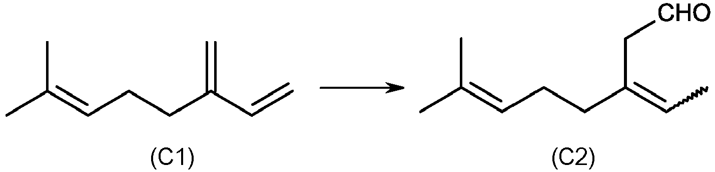

Für verschiedene Anwendungen, beispielsweise für die Synthese von Aromachemikalien (Riech- und Geschmacksstoffen), ist erwünscht, dass die Hydroformylierung mit einer anderen Regioselektivität als nach den bisher bekannten Verfahren verläuft, wobei eine möglichst hohe Ausbeute an den Hydroformylierungsprodukten in der delta-Position erzielt werden soll. Das angestrebte Hauptprodukt beispielsweise bei der Hydroformylierung von Isopren (B1) wäre dann das 4-Methylpent-4-enal (B3) 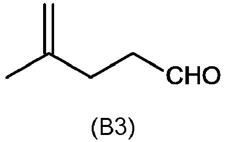 und das angestrebte Hauptprodukt bei der Hydroformylierung von Myrcen (C1) wäre dann das 8-Methyl-4-methylen-non-7-enal (C3)

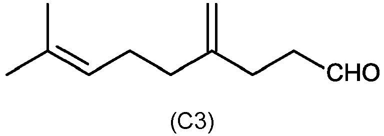

Ein weiteres interessantes Substrat für die regioselektive Hydroformylierung in der delta-Position ist das trans-ß-Farnesen ((6*E*)-7,11-Dimethyl-3-methylen-1,6,10-dodecatrien) (D):

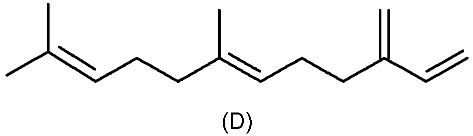

Es besteht somit Bedarf an einem Katalysatorsystem, das die regioselektive Hydroformylierung von 2-substituierten Butadienen in der delta-Position ohne Isomerisierung der zweiten Doppelbindung ermöglicht. Mit anderen Worten, soll möglichst nur die Vinylgruppe -CH=CH₂ der Hydroformylierung unterworfen werden. H. V. Barros, C. C. Guimaraes, E. N. dos Santos und E. V. Gusevskaya beschreiben in Organometallics, 2007, 26, 2211-2218 die Hydroformylierung von Isopren. Bei Verwendung von Rh/Triphenylphosphin als Katalysator werden 3-Methyl-pent-3-enal und 3-Methyl-pent-2-enal als Hauptprodukte beschrieben. Bei Verwendung von Rh/Diphosphin-Systemen, z. B. 1,2-Bis(diphenylphosphino)ethan, 1,3-Bis(diphenylphosphino)propan und 1,4-Bis(diphenylphosphino)butan ist das Hauptprodukt 3-Methyl-pent-2-enal, als Nebenprodukt werden u. a. bis zu 35 % 4-Methylpent-4-enal gefunden.

H. V. Barros, J. G. da Silva, C. C. Guimaraes, E. N. dos Santos und E. V. Gusevskaya beschreiben in Organometallics, 2008, 27, 4523-4531 die Hydroformylierung von β-Myrcen unter Verwendung von Rh/PPh₃ und Rh/PCy₃ als Katalysator. Als Hauptprodukt wird bei Umsätzen von bis zu 98 % und Selektivitäten von 77 % 3-Ethyliden-7-methyl-oct-6-enal erhalten.

In C. M. Foca, H. J. V. Barros, E. N. dos Santos, E. V. Gusevskaya, J. C. Baylon, New J. Chem., 2003, 27, 533-539 wird die Hydroformylierung von β-Myrcen mit Pt/Sn-Katalysatoren und Rhodium-Katalysatoren beschrieben. Bei der Verwendung von Pt/Sn als Katalysator wird unabhängig vom eingesetzten Phosphorliganden das Hydroformylierungsprodukt in der delta-Position, d. h. das 8-Methyl-4-methylen-non-7-enal, als Hauptprodukt mit einer Selektivität von bis zu 74 % gebildet. Die Ausbeuten liegen jedoch maximal bei 40,7 %. Weiterhin wird in diesem Dokument über den Einsatz von Rh-Phosphin-, Diphosphin- und Rh-Diphosphit-Katalysatorsystemen berichtet. Diese besitzen jedoch nicht dieselbe Leistung bei der Herstellung von 8-Methyl-4-methylen-non-7-enal wie die beschriebenen Pt/Sn-Katalysatoren. Nachteilig an den eingesetzten Pt/Sn-Katalysatoren ist, dass sie aufgrund ihres Chlorgehalts zu Korrosionsproblemen führen und daher für einen Einsatz in technischem Maßstab weniger geeignet sind. Es besteht daher Bedarf an einem Rhodium-Katalysatorsystem, das die Hydroformylierung von β-Myrcen (und vergleichbaren Olefinen) zumindest mit der gleichen Regioselektivität wie Pt/Sn-Katalysatoren ermöglicht.

In der WO 2010/033976 wird die Herstellung von Detergenzalkoholgemischen aus mehrfach verzweigten Polyolefinen beschrieben. Als mögliche Polyolefin-Ausgangsmaterialien werden unter anderem β-Myrcen und β-Farnesen genannt. Die Polyolefine werden einer Hydroformylierung und anschließenden Hydrierung zu mehrfach verzweigten Alkoholen unterzogen. In Synthesebeispiel III wird β-Farnesen unter Verwendung eines Rh/Xantphos/Triphenylphosphin-Katalysatorsystems hydroformyliert und das Reaktionsprodukt anschließend hydriert. Im Reaktionsprodukt liegen 39 % 4,8,12-Trimethyl-tridecan-1-ol und 34 % 3-Ethyl-7,11-Dimethyl-dodecan-1-ol vor; der Austrag der Hydroformylierung wurde nicht untersucht.

Der vorliegenden Erfindung liegt daher zum einen die Aufgabe zugrunde, ein Verfahren zur regioselektiven Hydroformylierung von 2-substituierten Butadien-Systemen zur Verfügung zu stellen, bei dem ein Produktgemisch erhalten wird, welches als Hauptprodukt das Produkt der Hydroformylierung in der delta-Position ohne Isomerisierung der zweiten Doppelbindung enthält. Mit anderen Worten soll das eingesetzte Katalysatorsystem eine möglichst selektive Hydroformylierung der Vinyl-Doppelbindung in hoher Ausbeute ermöglichen. In einer speziellen Ausführung liegt der Erfindung die Aufgabe zugrunde, ein Verfahren zur regioselektiven Hydroformylierung von trans-β-Farnesen zur Verfügung zu stellen, bei dem ein Produktgemisch erhalten wird, welches als Hauptprodukt (7E)-8,12-Dimethyl-4-methylen-trideca-7,11-dienal enthält. Dabei soll das eingesetzte Katalysatorsystem auf Rhodium basieren und möglichst keine korrosiven Liganden aufweisen.

(7E)-8,12-Dimethyl-4-methylen-trideca-7,11-dienal ist ein wichtiger Synthesebaustein und ein potentiell wichtiges Zwischenprodukt bei der Synthese von (3*E*,7*E*)-Homofarnesol:

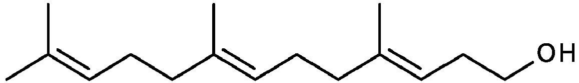

In der Literatur sind verschiedene Verfahren zur Herstellung von (3*E*,7*E*)-Homofarnesol beschrieben:
Es ist prinzipiell möglich, stereoisomerenreines (3*E*,7*E*)-Homofarnesol, ausgehend von (*E*,*E*)-Farnesol über (*E*, *E*)-Farnesal, C1-Verlängerung nach Wittig mit Methylentriphenylphosphoran und anschließende terminale Hydroborierung des konjugierten Diens herzustellen. Diese Synthese ist allerdings sehr aufwändig und weder technisch noch ökonomisch ein sinnvoller Zugang zu (3*E*,7*E*)-Homofarnesol.

A. F. Barrero et al., J. Org. Chem. 1996, 61, 2215 (2) beschreibt die Synthese des (3*E*,7*E*)-Homofarnesols über folgende Reaktionsschritte: a) Destillative Trennung von (*E*/*Z*)-Nerolidol, b) Umsetzung von (*E*)-Nerolidol mit Dimethylformamid-dimethylacetal (DMFDMA) in einer Büchi-Umlagerung zu den korrespondierenden (3*E*/*Z*, 7*E*)-C16-Amiden, c) Flashchromatographische Trennung der stereoisomeren Amide und d) Reduktion des (3*E*,7*E*)-Amids zum korrespondierenden (3*E*,7*E*)-Homofarnesol mit Lithium-triethylborhydrid.

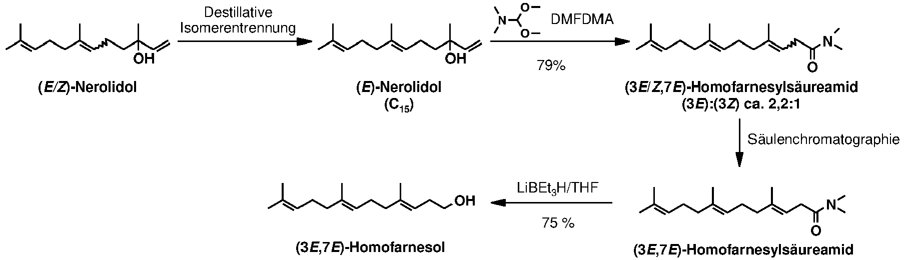

Nachteile dieser Route sind die moderaten Ausbeuten und die erforderliche FlashChromatographie zur Trennung der Stereoisomeren.

Die WO 92/06063 beschreibt ein Verfahren zur Herstellung von α,β,γ-ungesättigten Carbonsäuren durch Carbonylierung der entsprechenden allylischen Alkohole, z. B. die Carbonylierung von (*E*)-Nerolidol unter Zusatz katalytischer Mengen von Palladium(II)-chlorid. 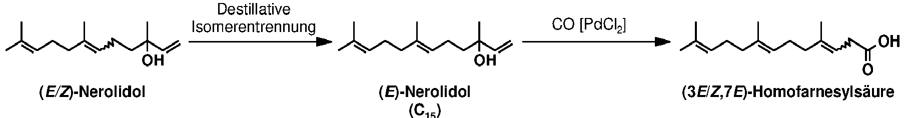 Beschrieben ist weiterhin die Reduktion des so erhaltenen Carbonylierungsprodukts zu Homofarnesol oder Monocyclohomofarnesol und die säurekatalysierte Cyclisierung unter Erhalt von 3a,6,6,9a-Tetramethyldodecahydronaphtho-[2,1-b]-furan, einen ambraartigen Duftstoff. Nachteilig an diesem Verfahren ist der Einsatz der relativ teuren Palladiumhalogenide. Des Weiteren findet die Carbonylierungsreaktion bei hohen CO-Drücken von ca. 70 bar statt.

Die WO 2013/156398 beschreibt ein Verfahren zur Herstellung von Verbindungen der allgemeinen Formel (E) 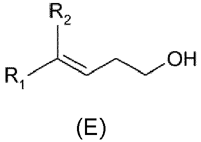 worin
R₁ für einen geradkettigen oder verzweigten, gegebenenfalls ein- oder mehrfach ungesättigten Hydrocarbylrest steht, und R₂ für H oder C₁-C₆-Alkyl steht, wobei man
a) eine Carbonylverbindung der Formel (F) 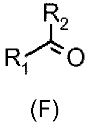 worin R₁ und R₂ die oben angegebenen Bedeutungen besitzen,
   mittels Wittig-Olefinierung zu einem Cyclopropan der allgemeinen Formel (G) 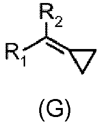 worin R₁ und R₂ die oben angegebenen Bedeutungen besitzen,
   umsetzt,
b) das Cyclopropan der Formel (G) unter Ringöffnung zu einer Verbindung der Formel (H) 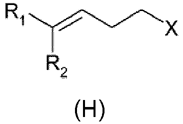 worin R₁ und R₂ die oben angegebenen Bedeutungen besitzen und X für Halogen oder O-R' steht, worin R' für H, Acyl, Tf-Acetyl oder SO₂-R" steht, worin R" für Alkyl oder Aryl steht;
   umsetzt und
c) die Verbindung der allgemeinen Formel (H) in die Verbindung der allgemeinen Formel (E) überführt.

Beschrieben ist unter anderem die Herstellung von Homofarnesol, insbesondere (3E,7E)-Homofarnesol aus Geranylaceton (d. h. einem C₁₃-Baustein) und Cyclopropylphosphoniumsalz (d. h. einem C₃-Baustein) in einer Wittig-Reaktion. Das (*E*)-C₁₆-Cyclopropan kann in Gegenwart einer Säure, z. B. einer Lewis-Säure wie AlCl₃ oder BF₃*Et₂O, und eines Nukleophils in regio- und stereoselektiver Weise zu Homofarnesylderivaten geöffnet werden. Anschließend kann das Homofarnesylchlorid durch Acetat-Substitution und Hydrolyse in das Homofarnesol überführt werden. Alternativ kann das Homofarnesol ausgehend vom Homofarnesylchlorid über das Formiat und anschließende Hydrolyse dargestellt werden. Auch die US 2013/0273619 A1 beschreibt ein Verfahren zur Herstellung von Homofarnesol, wobei Homofarnesylchlorid zu Homofarnesol umgesetzt wird.

Die zuvor beschriebenen Synthesen zur Herstellung von (3*E*,7*E*)-Homofarnesol sind insgesamt aufwändig, da sie mehrere Syntheseschritte unter Einsatz kostspieliger Reagenzien und/oder drastischer Reaktionsbedingungen erfordern. Der vorliegenden Erfindung liegt daher die weitere Aufgabe zugrunde, ein Verfahren zur Synthese von (3*E*,7*E*)-Homofarnesol unter Verwendung von (7E)-8,12-Dimethyl-4-methylen-trideca-7,11-dienal als Zwischenprodukt zur Verfügung zu stellen, das die zuvor beschriebenen Nachteile vermeidet.

Homofarnesol ist seinerseits ein wichtiges Zwischenprodukt von Syntheseverfahren zur Herstellung von Ambrox®. Insbesondere liefert die Cyclisierung des (3*E*,7*E*)-Homofarnesols diastereomerenreines bzw. enantiomerenreines Ambrox®. Ambrox® ist die Handelsbezeichnung für die enantiomerenreine Verbindung (-)-Ambrox (3aR,5aS,9aS,9bR)-3a,6,6,9a-tetramethyldodecahydronaphtho[2,1-b]furan, ein begehrter Riechstoff. Natürlich vorkommendes (-)-Ambrox ist der olfaktorisch bedeutsamste Inhaltsstoff von Ambra, einem Verdauungsprodukt von Pottwalen.

Die Cyclisierung des (3*E*,7*E*)-Homofarnesols zum Ambrox ist als solche bekannt, wobei sowohl enzymatische als auch chemische Cyclisierungen beschrieben sind.

So ist beispielsweise die enzymatische Cyclisierung mittels Squalen-Hopen-Cyclase aus der WO 2010/139719 bekannt.

Chemische Cyclisierungsreaktionen unter Verwendung einer Supersäure (Fluorsulfonsäure in 2-Nitropropan) sind z. B. aus P. F. Vlad et al. Khimiya Geterotsiklicheskikh Soedinenii, Engl. Transl. 1991, 746 bekannt. Weitere Verfahren umfassen die enantioselektive Polyencyclisierung von Homofarnesyltriethylsilylether in Gegenwart von O-(o-Fluorbenzyl)-binol und SnCl₄, wie von H. Yamamoto et al. J. Am. Chem. Soc. 2002, 3647 beschrieben.

Der vorliegenden Erfindung liegt daher die weitere Aufgabe zugrunde, ein Verfahren zur Synthese von Ambrox unter Verwendung von (7E)-8,12-Dimethyl-4-methylentrideca-7,11-dienal und von (3*E*,7*E*)-Homofarnesol als Zwischenstufen zur Verfügung zu stellen.

Überraschenderweise wurde jetzt gefunden, dass mit Rhodiumkatalysatoren mit speziellen Phosphorchelatverbindungen als Liganden die regioselektive Hydroformylierung von 2-substituierten Butadien-Systemen gelingt, wobei ein Produktgemisch erhalten wird, welches als Hauptprodukt das Produkt der Hydroformylierung der Vinyl-Doppelbindung ohne Isomerisierung der zweiten Doppelbindung enthält. Enthalten die zur Hydroformylierung eingesetzten Edukte neben dem Butadien-System weitere innenliegende Doppelbindungen, so werden diese im Wesentlichen weder hydroformyliert noch isomerisiert. Die als Liganden der Hydroformylierungskatalysatoren eingesetzten Phosphorchelatverbindungen weisen starre Rückgrate auf, d. h. die verbrückende Gruppe, an die die Phosphoratome (in der Regel über ein Sauerstoffatom) gebunden sind, ist nicht zur freien Drehbarkeit befähigt. Bevorzugt liegt zudem der Bisswinkel der Phosphorchelatliganden bei etwa 120°. Mit diesen Rhodiumkatalysatoren mit speziellen Phosphorchelatverbindungen als Liganden gelingt in vorteilhafter Weise die Hydroformylierung von trans-beta-Farnesen, wobei als Hauptprodukt (7E)-8,12-Dimethyl-4-methylen-trideca-7,11-dienal erhalten wird. Dieses lässt sich erfindungsgemäß weiter zum (-)-Ambrox umsetzen.

### ZUSAMMENFASSUNG DER ERFINDUNG

Ein erster Gegenstand der Erfindung ist ein Verfahren zur Herstellung von Verbindungen der allgemeinen Formel (I) und von Folgeprodukten davon, 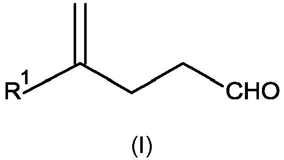 umfassend wenigstens einen Reaktionsschritt, bei dem man wenigstens eine Verbindung der allgemeinen Formel (II) 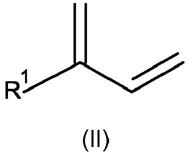 worin
R¹ für jeweils lineares oder verzweigte C₁-C₂₄-Alkyl oder C₂-C₂₄-Alkenyl mit 1, 2, 3 oder mehr als 3 C-C-Doppelbindungen steht,
einer Umsetzung mit Kohlenmonoxid und Wasserstoff in Gegenwart eines Hydroformylierungskatalysators unterzieht, der einen Rhodiumkomplex mit wenigstens einer Phosphorchelatverbindung als Liganden umfasst, wobei ein Reaktionsgemisch erhalten wird, das wenigstens 50,1 Gew.-%, bezogen auf das Gesamtgewicht des Reaktionsgemischs, wenigstens einer Verbindung der allgemeinen Formel (I) enthält. Der Hydroformylierungskatalysator umfasst wenigstens eine Phosphorchelatverbindung der allgemeinen Formel (VI) 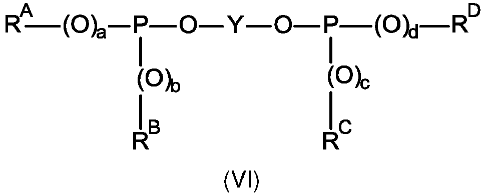 worin
R^{A}, R^{B}, R^{C} und R^{D} unabhängig voneinander für Alkyl, Cycloalkyl, Heterocycloalkyl, Aryl oder Hetaryl stehen,
   wobei die Alkylreste R^{A}, R^{B}, R^{C} und R^{D} unsubstituiert sind oder 1, 2, 3, 4 oder 5 gleiche oder verschiedene Substituenten tragen, die ausgewählt sind unter Cycloalkyl, Heterocycloalkyl, Aryl, Hetaryl, Alkoxy, Cycloalkoxy, Heterocycloalkoxy, Aryloxy, Hetaryloxy, Hydroxy, Mercapto, Polyalkylenoxid, Polyalkylenimin, COOH, Carboxylat, SO₃H, Sulfonat, NE¹E², NE¹E²E³⁺X⁻, Halogen, Nitro, Formyl, Acyl und Cyano, worin E¹, E² und E³ jeweils gleiche oder verschiedene Reste, ausgewählt unter Wasserstoff, Alkyl, Cycloalkyl, oder Aryl bedeuten und X- für ein Anionäquivalent steht,
   und wobei die Cycloalkyl-, Heterocycloalkyl-, Aryl- und Hetarylreste R^{A}, R^{B}, R^{C} und R^{D} jeweils unsubstituiert sind oder 1, 2, 3, 4 oder 5 gleiche oder verschiedene Substituenten tragen, die ausgewählt sind unter Alkyl und den zuvor für die Alkylreste R^{A}, R^{B}, R^{C} und R^{D} genannten Substituenten,
   oder
R^{A} und R^{B} und/oder R^{C} und R^{D} zusammen mit dem Phosphoratom und, falls vorhanden, den Sauerstoffatomen, an die sie gebunden sind, für einen 5- bis 8-gliedrigen Heterocyclus stehen, der gegebenenfalls zusätzlich ein-, zwei- oder dreifach mit Cycloalkyl, Heterocycloalkyl, Aryl oder Hetaryl anelliert ist, wobei der Heterocyclus und, falls vorhanden, die anellierten Gruppen unabhängig voneinander unsubstituiert sind oder einen, zwei, drei oder vier gleiche oder verschiedene Substituenten tragen, die ausgewählt sind unter Alkyl, Cycloalkyl, Heterocycloalkyl, Aryl, Hetaryl, Hydroxy, Mercapto, Polyalkylenoxid, Polyalkylenimin, Alkoxy, Halogen, COOH, Carboxylat, SO₃H, Sulfonat, NE⁴E⁵, NE⁴E⁵E⁶⁺X⁻, Nitro, Alkoxycarbonyl, Formyl, Acyl und Cyano, worin E⁴, E⁵ und E⁶ jeweils gleiche oder verschiedene Reste, ausgewählt unter Wasserstoff, Alkyl, Cycloalkyl und Aryl bedeuten und X⁻ für ein Anionäquivalent steht,
a, b, c und d unabhängig voneinander für 0 oder 1 stehen,
Y ausgewählt ist unter Gruppen der Formeln VII.a oder VII.b 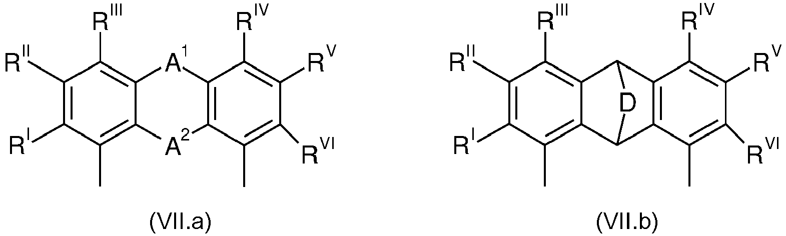
worin
R^{I}, R^{II}, R^{III}, R^{IV}, R^{V} und R^{VI} unabhängig voneinander für Wasserstoff, Alkyl, Cycloalkyl, Heterocycloalkyl, Aryl, Hetaryl, Hydroxy, Mercapto, Polyalkylenoxid, Polyalkylenimin, Alkoxy, Halogen, SO₃H, Sulfonat, NE⁷E⁸, Alkylen-NE⁷E⁸, Trifluormethyl, Nitro, Alkoxycarbonyl, Carboxyl, Formyl, Acyl oder Cyano stehen, worin E⁷ und E⁸ jeweils gleiche oder verschiedene Reste, ausgewählt unter Wasserstoff, Alkyl, Cycloalkyl und Aryl bedeuten,
   wobei zwei benachbarte Reste R^{I} bis R^{VI} gemeinsam mit den Kohlenstoffatomen des Benzolkerns, an die sie gebunden sind, auch für ein kondensiertes Ringsystem mit 1, 2 oder 3 weiteren Ringen stehen können,
A¹ und A² unabhängig voneinander für O, S, SiR^{E}R^{F}, NR^{G} oder CR^{H}R^{K} stehen, wobei RE, R^{F}, R^{G}, R^{H} und R^{K} unabhängig voneinander für Wasserstoff, Alkyl, Cycloalkyl, Heterocycloalkyl, Aryl oder Hetaryl stehen,
D für eine zweibindige Brückengruppe der allgemeinen Formel 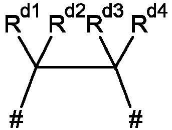
steht, worin
# jeweils für eine Bindungsstelle an das 9,10-Dihydroanthracengerüst steht,
R^{d1}, R^{d2}, R^{d3} und R^{d4} unabhängig voneinander für Wasserstoff, Alkyl, Cycloalkyl, Aryl, Halogen, Trifluormethyl, Carboxyl, Carboxylat oder Cyano stehen,
   wobei R^{d1} auch gemeinsam mit R^{d3} für den Bindungsanteil einer Doppelbindung zwischen den beiden Kohlenstoffatomen, an die R^{d1} und R^{d3} gebunden sind, stehen kann, und/oder R^{d2} und R^{d4} gemeinsam mit den Kohlenstoffatomen, an die sie gebunden sind, auch für einen 4- bis 8-gliedrigen Carbo- oder Heterocyclus stehen können, der gegebenenfalls zusätzlich ein-, zwei- oder dreifach mit Cycloalkyl, Heterocycloalkyl, Aryl oder Hetaryl anelliert ist, wobei der Carbo- oder Heterocyclus und, falls vorhanden, die anellierten Gruppen unabhängig voneinander unsubstituiert sind oder je einen, zwei, drei oder vier gleiche oder verschiedene Substituenten tragen, die ausgewählt sind unter Alkyl, Cycloalkyl, Heterocycloalkyl, Aryl, Hetaryl, COOR^{d5}, COO⁻M⁺, SO₃R^{d5}, SO₃⁻M⁺, NE⁹E¹⁰, Alkylen-NE⁹E¹⁰, NE⁹E¹⁰E¹¹⁺ X⁻, Alkylen-NE⁹E¹⁰E¹¹⁺ X⁻, OR^{d6}, SR^{d6}, (CHR^{f}CH₂O)_{y}R^{d6}, (CH₂N(E⁹))_{y}R^{d6}, (CH₂CH₂N(E9))_{y}R^{d6}, Halogen, Trifluormethyl, Nitro, Formyl, Acyl oder Cyano, stehen, worin
   R^{d5}, E⁹, E¹⁰ und E¹¹ jeweils gleiche oder verschiedene Reste, ausgewählt unter Wasserstoff, Alkyl, Cycloalkyl oder Aryl bedeuten,
   R^{d6} für Wasserstoff, Methyl oder Ethyl steht,
   M⁺ für ein Kationäquivalent steht,
   X⁻ für ein Anionäquivalent steht, und
   y für eine ganze Zahl von 1 bis 120 steht.

Ein weiterer Gegenstand der Erfindung ist ein Verfahren zur Hydroformylierung, bei dem man trans-β-Farnesen einer Umsetzung mit Kohlenmonoxid und Wasserstoff in Gegenwart eines wie oben beschriebenen Hydroformylierungskatalysators unterzieht, der einen Rhodiumkomplex mit wenigstens einer Phosphorchelatverbindung als Liganden umfasst, wie zuvor und im Folgenden definiert, wobei ein Reaktionsgemisch erhalten wird, das wenigstens 50,1 Gew.-%, bezogen auf das Gesamtgewicht des Reaktionsgemischs, (7E)-8,12-Dimethyl-4-methylen-trideca-7,11-dienal enthält.

Ein weiterer Gegenstand der Erfindung ist ein Verfahren, bei dem man
a) wenigstens eine Verbindung der allgemeinen Formel (II) 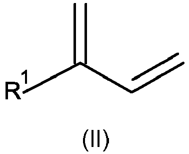 worin
   - R¹: für jeweils lineares oder verzweigte C₁-C₂₄-Alkyl oder C₂-C₂₄-Alkenyl mit 1, 2, 3 oder mehr als 3 C-C-Doppelbindungen steht,
   einer Umsetzung mit Kohlenmonoxid und Wasserstoff in Gegenwart eines wie oben beschriebenen Hydroformylierungskatalysators unterzieht, der einen Rhodiumkomplex mit wenigstens einer Phosphorchelatverbindung als Liganden umfasst, wobei ein Reaktionsgemisch erhalten wird, das wenigstens 50,1 Gew.-%, bezogen auf das Gesamtgewicht des Reaktionsgemischs, wenigstens einer Verbindung der allgemeinen Formel (I) enthält,
b) das in Schritt a) erhaltene Reaktionsgemisch oder eine an wenigstens einer Verbindung der allgemeinen Formel (I) angereicherte Fraktion daraus einer Hydrierung unterzieht, wobei ein Reaktionsgemisch erhalten wird, das wenigstens eine Verbindung der allgemeinen Formel (III) 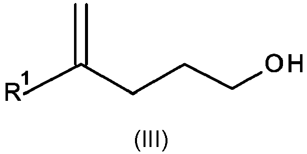 enthält.

Ein weiterer Gegenstand der Erfindung ist ein Verfahren, bei dem man
a) wenigstens eine Verbindung der allgemeinen Formel (II) 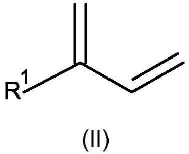 worin
   - R¹: für jeweils lineares oder verzweigte C₁-C₂₄-Alkyl oder C₂-C₂₄-Alkenyl mit 1, 2, 3 oder mehr als 3 C-C-Doppelbindungen steht,
   einer Umsetzung mit Kohlenmonoxid und Wasserstoff in Gegenwart eines wie oben beschriebenen Hydroformylierungskatalysators unterzieht, der einen Rhodiumkomplex mit wenigstens einer Phosphorchelatverbindung als Liganden umfasst, wobei ein Reaktionsgemisch erhalten wird, das wenigstens 50,1 Gew.-%, bezogen auf das Gesamtgewicht des Reaktionsgemischs, wenigstens einer Verbindung der allgemeinen Formel (I) enthält,
b) das in Schritt a) erhaltene Reaktionsgemisch oder eine an wenigstens einer Verbindung der allgemeinen Formel (I) angereicherte Fraktion daraus einer Hydrierung unterzieht, wobei ein Reaktionsgemisch erhalten wird, das wenigstens eine Verbindung der allgemeinen Formel (III) 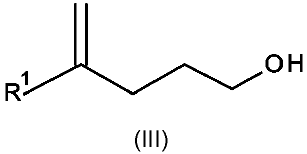 enthält,
c) die wenigstens eine Verbindung der allgemeinen Formel (III) einer zumindest teilweisen Isomerisierung unter Erhalt einer Verbindung der allgemeinen Formel (IV) 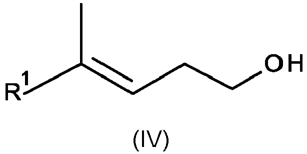 unterzieht.

Ein weiterer Gegenstand der Erfindung ist ein Verfahren zur Herstellung von (-)-Ambrox (V) 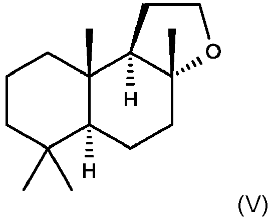 bei dem man
a1) (6*E*)-7,11-Dimethyl-3-methylidenedodeca-1,6,10-trien (II.1) 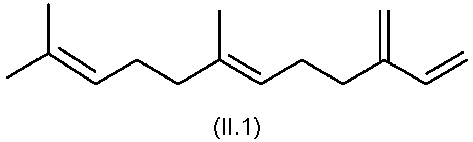 einer Umsetzung mit Kohlenmonoxid und Wasserstoff in Gegenwart eines Hydroformylierungskatalysators unterzieht, der einen Rhodiumkomplex mit wenigstens einer Phosphorchelatverbindung als Liganden umfasst, wobei ein Reaktionsgemisch erhalten wird, das wenigstens 50,1 Gew.-%, bezogen auf das Gesamtgewicht des Reaktionsgemischs, (7E)-8,12-Dimethyl-4-methylen-trideca-7,11-dienal (1.1) 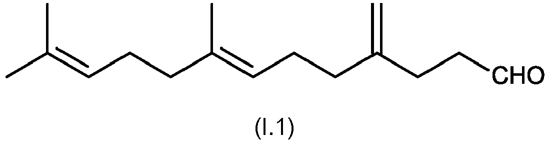 enthält,
b1) das in Schritt a) erhaltene Reaktionsgemisch oder eine an wenigstens einer Verbindung der allgemeinen Formel (I.1) angereicherte Fraktion daraus einer Hydrierung unterzieht, wobei ein Reaktionsgemisch erhalten wird, das die Verbindung (111.1) 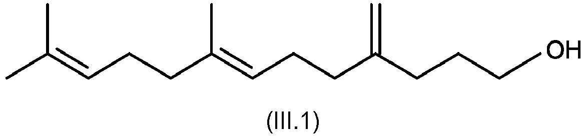 enthält,
c1) die Verbindung (III.1) einer zumindest teilweisen Isomerisierung unter Erhalt von (3*E*,7*E*)-Homofarnesol (IV.1) 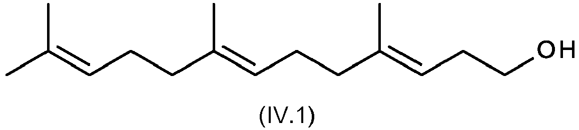 unterzieht,
d1) das (3*E*,7*E*)-Homofarnesol (IV.1) einer Cyclisierung unter Erhalt von (-)-Ambrox (V) unterzieht.

### BESCHREIBUNG DER ERFINDUNG

In den Verbindungen der allgemeinen Formel (I.2) 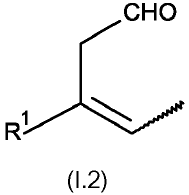 soll die geschlängelte Bindung andeuten, dass es sich um das reine cis-Isomer, das reine trans-Isomer oder ein beliebiges cis-/trans-Gemisch handeln kann.

Die folgenden Angaben zu geeigneten und bevorzugten Ausführungsformen der Hydroformylierung wenigstens einer Verbindung der allgemeinen Formel (II) unter Erhalt eines Reaktionsgemischs, das wenigstens 50,1 Gew.-% wenigstens einer Verbindung der allgemeinen Formel (I) enthält, gelten gleichermaßen für die Ausführungsform der Erfindung, nach der die Verbindung (I) das Endprodukt darstellt als auch die Ausführungsformen, nach denen die Verbindung (I) ein Zwischenprodukt darstellt. Wenn die Verbindung (I) ein Zwischenprodukt ist, wird dieser Reaktionsschritt auch als "Schritt a)" bezeichnet.

Die folgenden Angaben zu geeigneten und bevorzugten Ausführungsformen beim Reaktionsschritt a) gelten analog für den Reaktionsschritt a1), sofern keine speziellen Angaben gemacht werden. Die folgenden Angaben zu geeigneten und bevorzugten Ausführungsformen beim Reaktionsschritt b) gelten analog für den Reaktionsschritt b1), sofern keine speziellen Angaben gemacht werden. Die folgenden Angaben zu geeigneten und bevorzugten Ausführungsformen beim Reaktionsschritt c) gelten analog für den Reaktionsschritt c1), sofern keine speziellen Angaben gemacht werden.

### Hydroformylierung (Schritt a))

Vorzugsweise weisen die Verbindungen der Formel (I) nur eine Vinylgruppe, d. h. nur eine Gruppe der Formel -CH=CH₂ im Molekül auf.

Besonders bevorzugt sind die Verbindungen der Formel (II) ausgewählt unter Isopren, β-Myrcen und β-Farnesen. Eine besonders bevorzugte Verbindung der Formel (II) ist (6*E*)-7,11-Dimethyl-3-methylidendodeca-1,6,10-trien (= trans-β-Farnesen).

In einer speziellen Ausführungsform wird in dem erfindungsgemäßen Verfahren β-Farnesen einer Hydroformylierung unterzogen.

Das erfindungsgemäße Verfahren ermöglicht die Hydroformylierung von Verbindungen der allgemeinen Formel (I) mit hoher Ausbeute und mit hoher Selektivität.

Der Umsatz der Verbindungen der allgemeinen Formel (I) beträgt im Allgemeinen wenigstens 65 %, vorzugsweise wenigstens 80 %, besonders wenigstens 85 %, insbesondere wenigstens 90 %, bezogen auf die eingesetzte Menge an Verbindungen der allgemeinen Formel (I). Mit ausgewählten Liganden kann auch ein höherer Umsatz erzielt werden.

Die Selektivität bezüglich der Verbindungen der allgemeinen Formel (11.1) beträgt im Allgemeinen wenigstens 55 %, vorzugsweise wenigstens 60 %, besonders wenigstens 65 %, insbesondere wenigstens 70 %, bezogen auf die umgesetzte Menge an Verbindungen der allgemeinen Formel (I). Mit ausgewählten Liganden kann auch eine höhere Selektivität erzielt werden.

Bevorzugt wird nach dem erfindungsgemäßen Verfahren ein Reaktionsgemisch erhalten wird, das wenigstens 55 Gew.-%, vorzugsweise wenigstens 60 %, insbesondere wenigstens 65 Gew.-%, bezogen auf das Gesamtgewicht des Reaktionsgemischs, wenigstens einer Verbindung der allgemeinen Formel (11.1) enthält. Mit ausgewählten Liganden kann auch ein höherer Umsatz erzielt werden.

Bevorzugt enthält das nach dem erfindungsgemäßen Verfahren erhaltene Reaktionsgemisch insgesamt höchstens 49,9 Gew.-%, bevorzugt höchstens 45 Gew.-%, insbesondere höchstens 40 Gew.-%, speziell höchstens 35 Gew.-%, an Verbindungen, die ausgewählt sind unter der Verbindung der allgemeinen Formel (I.2) 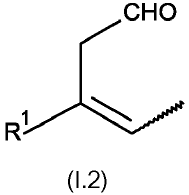 worin R¹ für jeweils lineares oder verzweigte C₁-C₂₄-Alkyl, oder C₂-C₂₄-Alkenyl mit 1, 2, 3 oder mehr als 3 C-C-Doppelbindungen steht, und gegebenenfalls weiteren von den Verbindungen (I) und (I.2) verschiedenen Verbindungen. Weitere von den Verbindungen (I) und (I.2) verschiedene Verbindungen können resultieren durch Hydroformylierung einer anderen Doppelbindung an Stelle von oder zusätzlich zu der endständigen Vinylgruppe, Isomerisierung wenigstens einer Doppelbindung, Hydrierung wenigstens einer Doppelbindung, Kombinationen aus wenigstens zwei dieser Reaktionen. Eine Übersicht über die bei der Hydroformylierung von Myrcen erhaltenen Produkte wird von C. M. Foca et al., New J. Chem., 2003, 27, 533-539 im Schema 1 auf Seite 533 gegeben.

Bevorzugt weist der eingesetzte Hydroformylierungskatalysator wenigstens eine zweizähnige Phosphorchelatverbindung als Liganden auf, die einen natürlichen Bisswinkel im Bereich von etwa 120° aufweisen. Der Ausdruck "natürlicher Bisswinkel (natural bite-angle) wird dabei fachüblich verstanden, wie er z. B. in P.W.N.M. van Leeuwen et al., Chem. Rev. 2000, 2741, definiert ist.

Erfindungsgemäß wird ein Katalysator eingesetzt, der als Metall Rhodium umfasst.

Im Folgenden umfasst der Ausdruck "Alkyl" geradkettige und verzweigte Alkylgruppen. Vorzugsweise handelt es sich dabei um geradkettige oder verzweigte C₁-C₂₀-Alkyl, bevorzugter Weise C₁-C₁₂-Alkyl-, besonders bevorzugt C₁-C₈-Alkyl- und ganz besonders bevorzugt C₁-C₆-Alkylgruppen. Beispiele für Alkylgruppen sind insbesondere
Methyl, Ethyl, Propyl, Isopropyl, n-Butyl, Isobutyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl- 2-methylpropyl, n-Heptyl, 2-Heptyl, 3-Heptyl, 2-Ethylpentyl, 1-Propylbutyl, n-Octyl, 2-Ethylhexyl, 2-Propylheptyl, Nonyl, Decyl.

Der Ausdruck "Alkyl" umfasst auch substituierte Alkylgruppen, welche im Allgemeinen 1, 2, 3, 4 oder 5, bevorzugt 1, 2 oder 3 und besonders bevorzugt 1 Substituenten, ausgewählt aus den Gruppen Cycloalkyl, Aryl, Hetaryl, Halogen, NE¹E², NE¹E²E³⁺, COOH, Carboxylat, SO₃H und Sulfonat, tragen können. Eine bevorzugte fluorierte Alkylgruppe ist Trifluormethyl. Der Ausdruck "Alkyl" umfasst auch Alkylgruppen, die durch ein oder mehrere, nicht benachbarte Sauerstoffatome unterbrochen sind, vorzugsweise Alkoxyalkyl.

Der Ausdruck "Alkylen" im Sinne der vorliegenden Erfindung steht für geradkettige oder verzweigte Alkandiyl-Gruppen mit vorzugsweise 1 bis 6 Kohlenstoffatomen. Dazu zählen Methylen (-CH₂-), Ethylen (-CH₂-CH₂-), n-Propylen (-CH₂-CH₂-CH₂-), Isopropylen (-CH₂-CH(CH₃)-), etc.

Der Ausdruck "Cycloalkyl" umfasst im Sinne der vorliegenden Erfindung unsubstituierte als auch substituierte Cycloalkylgruppen, vorzugsweise C₅-C₇-Cycloalkylgruppen, wie Cyclopentyl, Cyclohexyl oder Cycloheptyl, die im Falle einer Substitution, im Allgemeinen 1, 2, 3, 4 oder 5, bevorzugt 1, 2 oder 3 und besonders bevorzugt 1 Substituenten, ausgewählt aus den Gruppen Alkyl, Alkoxy und Halogen, tragen können.

Der Ausdruck "Heterocycloalkyl" im Sinne der vorliegenden Erfindung umfasst gesättigte oder teilweise ungesättigte cycloaliphatische Gruppen mit im Allgemeinen 4 bis 7, vorzugsweise 5 oder 6 Ringatomen, in denen 1, 2, 3 oder 4 der Ringkohlenstoffatome durch Heteroatome, vorzugsweise ausgewählt aus den Elementen Sauerstoff, Stickstoff und Schwefel, ersetzt sind und die gegebenenfalls substituiert sein können. Im Falle einer Substitution weisen diese heterocycloaliphatischen Gruppen bevorzugt 1, 2 oder 3, besonders bevorzugt 1 oder 2, insbesondere 1 Substituenten auf. Diese sind vorzugsweise ausgewählt aus Alkyl, Cycloalkyl, Aryl, COOR (R = H, Alkyl, Cycloalkyl, Aryl), COO-M⁺ und NE¹E², bevorzugt Alkyl. Beispielhaft für solche heterocycloaliphatischen Gruppen seien Pyrrolidinyl, Piperidinyl, 2,2,6,6-Tetramethylpiperidinyl, Imidazolidinyl, Pyrazolidinyl, Oxazolidinyl, Morpholidinyl, Thiazolidinyl, Isothiazolidinyl, Isoxazolidinyl, Piperazinyl, Tetrahydrothiophenyl, Tetrahydrofuranyl, Tetrahydropyranyl, Dioxanyl genannt.

Der Ausdruck "Aryl" umfasst im Sinne der vorliegenden Erfindung sowohl unsubstituierte als auch substituierte Arylgruppen, und steht vorzugsweise für Phenyl, Tolyl, Xylyl, Mesityl, Naphthyl, Fluorenyl, Anthracenyl, Phenanthrenyl oder Naphthacenyl, besonders bevorzugt für Phenyl oder Naphthyl. Diese Arylgruppen können im Falle einer Substitution im Allgemeinen 1, 2, 3, 4 oder 5, vorzugsweise 1, 2 oder 3 und besonders bevorzugt 1 Substituenten, tragen. Diese sind bevorzugt ausgewählt aus den Gruppen Alkyl, Alkoxy, Carboxyl, Carboxylat, Trifluormethyl, - SO₃H, Sulfonat, NE¹E², Alkylen-NE¹E², Nitro, Cyano oder Halogen. Ein bevorzugter fluorierter Arylrest ist Pentafluorphenyl.

Der Ausdruck "Hetaryl" umfasst im Sinne der vorliegenden Erfindung unsubstituierte oder substituierte, heterocycloaromatische Gruppen, vorzugsweise die Gruppen Pyridyl, Chinolinyl, Acridinyl, Pyridazinyl, Pyrimidinyl, Pyrazinyl, sowie die Untergruppe der "Pyrrolgruppe", wobei diese heterocycloaromatischen Gruppen im Falle einer Substitution im Allgemeinen 1, 2 oder 3 Substituenten, ausgewählt aus den Gruppen Alkyl, Alkoxy, Carboxyl, Carboxylat, -SO₃H, Sulfonat, NE¹E², Alkylen-NE¹E², Trifluormethyl oder Halogen, tragen können.

Der Ausdruck "Pyrrolgruppe" steht im Sinne der vorliegenden Erfindung für eine Reihe unsubstituierter oder substituierter, heterocycloaromatischer Gruppen, die strukturell vom Pyrrolgrundgerüst abgeleitet sind und ein pyrrolisches Stickstoffatom im Heterocyclus enthalten, das kovalent mit anderen Atomen, beispielsweise einem Phosphoratom, verknüpft werden kann. Der Ausdruck "Pyrrolgruppe" umfasst somit die unsubstituierten oder substituierten Gruppen Pyrrolyl, Imidazolyl, Pyrazolyl, Indolyl, Purinyl, Indazolyl, Benzotriazolyl, 1,2,3-Triazolyl, 1,3,4-Triazolyl und Carbazolyl, die im Falle einer Substitution im Allgemeinen 1, 2 oder 3, vorzugsweise 1 oder 2, besonders bevorzugt 1 Substituenten, ausgewählt aus den Gruppen Alkyl, Alkoxy, Formyl, Acyl, Carboxyl, Carboxylat, -SO₃H, Sulfonat, NE¹E², Alkylen-NE¹E², Trifluormethyl oder Halogen, tragen können. Eine bevorzugte substituierte Indolylgruppe ist die 3-Methylindolylgruppe.

Dementsprechend umfasst der Ausdruck "Bispyrrolgruppe" im Sinne der vorliegenden Erfindung zweibindige Gruppen der Formel

Py-I-Py,

die zwei durch direkte chemische Bindung oder Alkylengruppen, -O-, -S-, Imino-, Silyl- oder Alkyliminogruppen vermittelte Verknüpfung, verbundene Pyrrolgruppen enthalten, wie die Bisindolyl-Gruppe der Formel 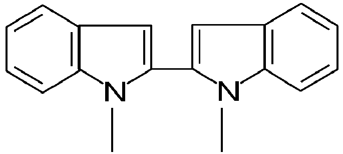 als Beispiel für eine Bispyrrolgruppe, die zwei direkt verknüpfte Pyrrolgruppen, in diesem Falle Indolyl, enthält, oder die Bispyrroldiylmethan-Gruppe der Formel 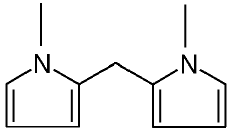 als Beispiel für eine Bispyrrolgruppe, die zwei über eine Methylengruppe verknüpfte Pyrrolgruppen, in diesem Falle Pyrrolyl, enthält. Wie die Pyrrolgruppen können auch die Bispyrrolgruppen unsubstituiert oder substituiert sein und im Falle einer Substitution pro Pyrrolgruppeneinheit im Allgemeinen 1, 2 oder 3, vorzugsweise 1 oder 2, insbesondere 1 Substituenten, ausgewählt aus Alkyl, Alkoxy, Carboxyl, Carboxylat, - SO₃H, Sulfonat, NE¹E², Alkylen-NE¹E², Trifluormethyl oder Halogen, tragen, wobei bei diesen Angaben zur Anzahl möglicher Substituenten die Verknüpfung der Pyrrolgruppeneinheiten durch direkte chemische Bindung oder durch die mittels der vorstehend genannten Gruppen vermittelte Verknüpfung nicht als Substitution betrachtet wird.

Carboxylat und Sulfonat stehen im Rahmen dieser Erfindung vorzugsweise für ein Derivat einer Carbonsäurefunktion bzw. einer Sulfonsäurefunktion, insbesondere für ein Metallcarboxylat oder -sulfonat, eine Carbonsäure- oder Sulfonsäureesterfunktion oder eine Carbonsäure- oder Sulfonsäureamidfunktion. Dazu zählen z. B. die Ester mit C₁-C₄-Alkanolen, wie Methanol, Ethanol, n-Propanol, Isopropanol, n-Butanol, sec.-Butanol und tert.-Butanol. Dazu zählen weiterhin die primären Amide und deren N-Alkyl- und N,N-Dialkylderivate.

Die obigen Erläuterungen zu den Ausdrücken "Alkyl", "Cycloalkyl", "Aryl", "Heterocycloalkyl" und "Hetaryl" gelten entsprechend für die Ausdrücke "Alkoxy", "Cycloalkoxy", "Aryloxy", "Heterocycloalkoxy" und "Hetaryloxy".

Der Ausdruck "Acyl" steht im Sinne der vorliegenden Erfindung für Alkanoyl- oder Aroylgruppen mit im Allgemeinen 2 bis 11, vorzugsweise 2 bis 8 Kohlenstoffatomen, beispielsweise für die Acetyl-, Propanoyl-, Butanoyl-, Pentanoyl-, Hexanoyl-, Heptanoyl-, 2-Ethylhexanoyl-, 2-Propylheptanoyl-, Benzoyl- oder Naphthoyl-Gruppe.

Die Gruppen NE¹E², NE⁴E⁵, NE⁷E⁸, NE⁹E¹⁰ und NE¹²E¹³ stehen vorzugsweise für N,N-Dimethylamino, N,N-Diethylamino, N,N-Dipropylamino, N,N-Diisopropylamino, N,N-Di-n-butylamino, N,N-Di-t.-butylamino, N,N-Dicyclohexylamino oder N,N-Diphenylamino.

Halogen steht für Fluor, Chlor, Brom und lod, bevorzugt für Fluor, Chlor und Brom.

M⁺ steht für ein Kationäquivalent, d. h. für ein einwertiges Kation oder den einer positiven Einfachladung entsprechenden Anteil eines mehrwertigen Kations. Das Kation M⁺ dient lediglich als Gegenion zur Neutralisation negativ geladener Substituentengruppen, wie der COO- oder der Sulfonat-Gruppe, und kann im Prinzip beliebig gewählt werden. Vorzugsweise werden deshalb Alkalimetall-, insbesondere Na⁺, K⁺-, Li⁺-Ionen oder Onium-Ionen, wie Ammonium-, Mono-, Di-, Tri-, Tetraalkylammonium-, Phosphonium-, Tetraalkylphosphonium- oder Tetraarylphosphonium-Ionen verwendet.

Entsprechendes gilt für das Anionäquivalent X⁻, das lediglich als Gegenion positiv geladener Substituentengruppen, wie den Ammoniumgruppen, dient und beliebig gewählt werden kann unter einwertigen Anionen und den einer negativen Einfachladung entsprechenden Anteilen eines mehrwertigen Anions. Geeignete Anionen sind z. B. Halogenid-Ionen X⁻, wie Chlorid und Bromid. Bevorzugte Anionen sind Sulfat und Sulfonat, z. B. SO₄²⁻, Tosylat, Trifluormethansulfonat und Methylsulfonat.

Die Werte für y stehen für eine ganze Zahl von 1 bis 120, vorzugsweise für eine ganze Zahl von 3 bis 120.

Kondensierte Ringsysteme können durch Anellierung verknüpfte (ankondensierte) aromatische, hydroaromatische und cyclische Verbindungen sein. Kondensierte Ringsysteme bestehen aus zwei, drei oder mehr als drei Ringen. Je nach der Verknüpfungsart unterscheidet man bei kondensierten Ringsystemen zwischen einer ortho-Anellierung, d. h. jeder Ring hat mit jedem Nachbarring jeweils eine Kante bzw. zwei Atome gemeinsam, und einer peri-Anellierung, bei der ein Kohlenstoffatom mehr als zwei Ringen angehört. Bevorzugt unter den kondensierten Ringsystemen sind ortho-kondensierte Ringsysteme.

Der Ausdruck Phosphorchelatverbindung bezeichnet im Rahmen der Erfindung speziell eine Verbindung, bei der wenigstens zwei phosphoratomhaltige Gruppen kovalent an ein und dasselbe Molekülgerüst gebunden sind. Die einzelnen Atome des Molekülgerüsts sind dabei ebenfalls über kovalente Bindungen miteinander verbunden.

Die als Hydroformylierungskatalysatoren eingesetzten Rhodiumkomplexe mit wenigstens einer Phosphorchelatverbindung als Liganden sind prinzipiell bekannt und z. B. in WO 01/58589, WO 02/083695, WO 02/22261, WO 03/018192, WO 2004/026803, WO 2005/009934, WO 2005/039762, WO 2005/063730 und DE 103 42 760 A1 beschrieben.

Bevorzugt umfasst der eingesetzte Hydroformylierungskatalysator wenigstens eine Phosphorchelatverbindung der allgemeinen Formel (VI) 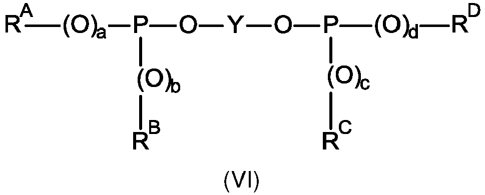 worin
R^{A}, R^{B}, R^{C} und R^{D} unabhängig voneinander für Alkyl, Cycloalkyl, Heterocycloalkyl, Aryl oder Hetaryl stehen, wobei die Alkylreste R^{A}, R^{B}, R^{C} und R^{D} unsubstituiert sind oder 1, 2, 3, 4 oder 5 gleiche oder verschiedene Substituenten tragen, die ausgewählt sind unter Cycloalkyl, Heterocycloalkyl, Aryl, Hetaryl, Alkoxy, Cycloalkoxy, Heterocycloalkoxy, Aryloxy, Hetaryloxy, Hydroxy, Mercapto, Polyalkylenoxid, Polyalkylenimin, COOH, Carboxylat, SO₃H, Sulfonat, NE¹E², NE¹E²E³⁺X-, Halogen, Nitro, Formyl, Acyl und Cyano, worin E¹, E² und E³ jeweils gleiche oder verschiedene Reste, ausgewählt unter Wasserstoff, Alkyl, Cycloalkyl, oder Aryl bedeuten und X⁻ für ein Anionäquivalent steht,
   und wobei die Cycloalkyl-, Heterocycloalkyl-, Aryl- und Hetarylreste R^{A}, R^{B}, R^{C} und R^{D} jeweils unsubstituiert sind oder 1, 2, 3, 4 oder 5 gleiche oder verschiedene Substituenten tragen, die ausgewählt sind unter Alkyl und den zuvor für die Alkylreste R^{A}, R^{B}, R^{C} und R^{D} genannten Substituenten,
   oder
R^{A} und R^{B} und/oder R^{C} und R^{D} zusammen mit dem Phosphoratom und, falls vorhanden, den Sauerstoffatomen, an die sie gebunden sind, für einen 5- bis 8-gliedrigen Heterocyclus stehen, der gegebenenfalls zusätzlich ein-, zwei- oder dreifach mit Cycloalkyl, Heterocycloalkyl, Aryl oder Hetaryl anelliert ist, wobei der Heterocyclus und, falls vorhanden, die anellierten Gruppen unabhängig voneinander unsubstituiert sind oder einen, zwei, drei oder vier gleiche oder verschiedene Substituenten tragen, die ausgewählt sind unter Alkyl, Cycloalkyl, Heterocycloalkyl, Aryl, Hetaryl, Hydroxy, Mercapto, Polyalkylenoxid, Polyalkylenimin, Alkoxy, Halogen, COOH, Carboxylat, SO₃H, Sulfonat, NE⁴E⁵, NE⁴E⁵E⁶⁺X⁻, Nitro, Alkoxycarbonyl, Formyl, Acyl und Cyano, worin E⁴, E⁵ und E⁶ jeweils gleiche oder verschiedene Reste, ausgewählt unter Wasserstoff, Alkyl, Cycloalkyl und Aryl bedeuten und X⁻ für ein Anionäquivalent steht,
a, b, c und d unabhängig voneinander für 0 oder 1 stehen,
Y ausgewählt ist unter Gruppen der Formeln VII.a oder VII.b 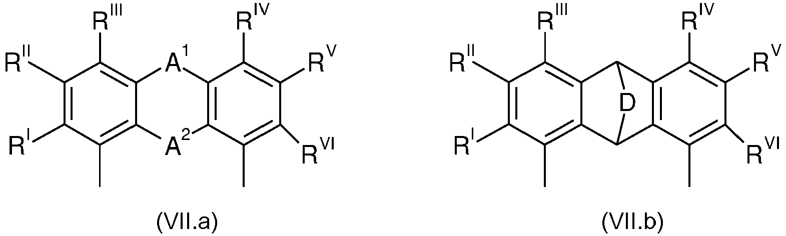
worin
R^{I}, R^{II}, R^{III}, R^{IV}, R^{V} und R^{VI} unabhängig voneinander für Wasserstoff, Alkyl, Cycloalkyl, Heterocycloalkyl, Aryl, Hetaryl, Hydroxy, Mercapto, Polyalkylenoxid, Polyalkylenimin, Alkoxy, Halogen, SO₃H, Sulfonat, NE⁷E⁸, Alkylen-NE⁷E⁸, Trifluormethyl, Nitro, Alkoxycarbonyl, Carboxyl, Formyl, Acyl oder Cyano stehen, worin E⁷ und E⁸ jeweils gleiche oder verschiedene Reste, ausgewählt unter Wasserstoff, Alkyl, Cycloalkyl und Aryl bedeuten,
   wobei zwei benachbarte Reste R^{I} bis R^{VI} gemeinsam mit den Kohlenstoffatomen des Benzolkerns, an die sie gebunden sind, auch für ein kondensiertes Ringsystem mit 1, 2 oder 3 weiteren Ringen stehen können,
A¹ und A² unabhängig voneinander für O, S, SiR^{E}R^{F}, NR^{G} oder CR^{H}R^{K} stehen, wobei R^{E}, R^{F}, R^{G}, R^{H} und R^{K} unabhängig voneinander für Wasserstoff, Alkyl, Cycloalkyl, Heterocycloalkyl, Aryl oder Hetaryl stehen,
D für eine zweibindige Brückengruppe der allgemeinen Formel 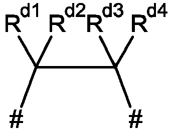
steht, worin
# jeweils für eine Bindungsstelle an das 9,10-Dihydroanthracengerüst steht,
R^{d1}, R^{d2}, R^{d3} und R^{d4} unabhängig voneinander für Wasserstoff, Alkyl, Cycloalkyl, Aryl, Halogen, Trifluormethyl, Carboxyl, Carboxylat oder Cyano stehen,
   wobei R^{d1} auch gemeinsam mit R^{d3} für den Bindungsanteil einer Doppelbindung zwischen den beiden Kohlenstoffatomen, an die R^{d1} und R^{d3} gebunden sind, stehen kann, und/oder R^{d2} und R^{d4} gemeinsam mit den Kohlenstoffatomen, an die sie gebunden sind, auch für einen 4- bis 8-gliedrigen Carbo- oder Heterocyclus stehen können, der gegebenenfalls zusätzlich ein-, zwei- oder dreifach mit Cycloalkyl, Heterocycloalkyl, Aryl oder Hetaryl anelliert ist, wobei der Carbo- oder Heterocyclus und, falls vorhanden, die anellierten Gruppen unabhängig voneinander unsubstituiert sind oder je einen, zwei, drei oder vier gleiche oder verschiedene Substituenten tragen, die ausgewählt sind unter Alkyl, Cycloalkyl, Heterocycloalkyl, Aryl, Hetaryl, COOR^{d5}, COO⁻M⁺, SO₃R^{d5}, SO₃⁻M⁺, NE⁹E¹⁰, Alkylen-NE⁹E¹⁰, NE⁹E¹⁰E¹¹⁺ X⁻, Alkylen-NE⁹E¹⁰E¹¹⁺ X⁻, OR^{d6}, SR^{d6}, (CHR^{f}CH₂O)_{y}R^{d6}, (CH₂N(E⁹))_{y}R^{d6}, (CH₂CH₂N(E⁹))_{y}R^{d6}, Halogen, Trifluormethyl, Nitro, Formyl, Acyl oder Cyano, stehen, worin
R^{d5}, E⁹, E¹⁰ und E¹¹ jeweils gleiche oder verschiedene Reste, ausgewählt unter Wasserstoff, Alkyl, Cycloalkyl oder Aryl bedeuten,
R^{d6} für Wasserstoff, Methyl oder Ethyl steht,
M⁺ für ein Kationäquivalent steht,
X⁻ für ein Anionäquivalent steht, und
y für eine ganze Zahl von 1 bis 120 steht.

Bevorzugt haben in den Verbindungen der Formel (VI) die Reste R^{A}, R^{B}, R^{C} und R^{D} alle die gleiche Bedeutung.

Bevorzugt steht die Brückengruppe Y für eine Gruppe der Formel VII. a, worin die Gruppen A¹ und A² ausgewählt sind aus den Gruppen O, S und CR^{H}R^{K}, insbesondere unter O, S, der Methylengruppe (R^{H}= R^{K} = H), der Dimethylmethylengruppe (R^{H} = R^{K} = CH₃), der Diethylmethylengruppe (R^{H}= R^{K} = C₂H₅), der Di-n-propyl-methylengruppe (R^{H} = R^{K} = n-Propyl) oder der Di-n-butylmethylengruppe (R^{H} = R^{K} = n-Butyl). Insbesondere sind solche Brückengruppen Y bevorzugt, in denen A¹ von A² verschieden ist, wobei A1 bevorzugt eine CR^{H}R^{K}-Gruppe und A2 bevorzugt eine O- oder S-Gruppe, besonders bevorzugt eine Oxagruppe O ist.

Weiterhin bevorzugt steht die Brückengruppe Y für eine Gruppe der Formel Vll.b, worin D für eine zweibindige Brückengruppe steht, die ausgewählt ist aus den Gruppen 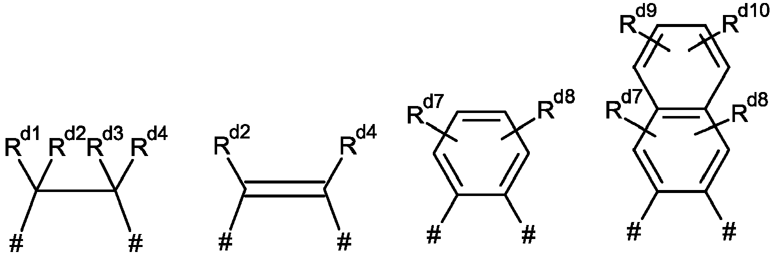 in denen R^{d1}, R^{d2}, R^{d3} und R^{d4} unabhängig voneinander für Wasserstoff, Alkyl, Cycloalkyl, Aryl, Halogen, Trifluormethyl, Carboxyl, Carboxylat oder Cyano stehen oder miteinander zu einer C₃-C₄-Alkylengruppe verbunden sind, und R^{d7}, R^{d8}, R^{d9} und R^{d10} unabhängig voneinander für Wasserstoff, Alkyl, Cycloalkyl, Aryl, Halogen, Trifluormethyl, COOH, Carboxylat, Cyano, Alkoxy, SO₃H, Sulfonat, NE⁹E¹⁰, Alkylen-NE⁹E¹⁰E¹¹⁺X⁻, Aryl oder Nitro stehen können. Vorzugsweise stehen die Gruppen für Wasserstoff, C₁-C₁₀-Alkyl oder Carboxylat und die Gruppen R^{d7}, R^{d8}, R^{d9} und R^{d10} für Wasserstoff, C₁-C₁₀-Alkyl, Halogen, insbesondere Fluor, Chlor oder Brom, Trifluormethyl, C₁-C₄-Alkoxy, Carboxylat, Sulfonat oder Aryl. Besonders bevorzugt stehen R^{d7}, R^{d8}, R^{d9} und R^{d10} für Wasserstoff.

Besonders bevorzugte Brückengruppen D sind die Ethylengruppe und die 1, 2-Phenylengruppe.

Eine besonders bevorzugte Brückengruppe D ist die Ethylengruppe -CH₂-CH₂-. Die Brückengruppen Y der Formel (VII.b) haben dann bevorzugt ein Triptycen-artiges Kohlenstoffgerüst.

In den Brückengruppen Y der Formel VII.a und Vll.b sind die Substituenten R^{I}, R^{II}, R^{III}, R^{IV}, R^{V} und R^{VI} vorzugsweise ausgewählt unter Wasserstoff, Alkyl, Alkoxy, Cycloalkyl, Heterocycloalkyl, Aryl und Hetaryl.

Nach einer ersten bevorzugten Ausführungsform stehen R^{I}, R^{II}, R^{III}, R^{IV}, R^{V} und R^{VI} alle für Wasserstoff.

Nach einer weiteren bevorzugten Ausführungsform stehen R^{II} und R^{V} unabhängig voneinander für C₁-C₄-Alkyl oder C₁-C₄-Alkoxy und R^{I}, R^{III}, R^{IV} und R^{VI} für Wasserstoff. Vorzugsweise sind R^{II} und R^{V} ausgewählt unter Methyl, Ethyl, Isopropyl, tert.-Butyl und Methoxy.

Nach einer weiteren bevorzugten Ausführungsform stehen R^{I} und R^{VI} unabhängig voneinander für C₁-C₄-Alkyl oder C₁-C₄-Alkoxy und R^{II}, R^{III}, R^{IV} und R^{V} für Wasserstoff. Vorzugsweise sind R^{I} und R^{VI} ausgewählt unter Methyl, Ethyl, Isopropyl, tert.-Butyl und Methoxy.

Insbesondere steht Y für eine Gruppe der Formel (VII.a), worin R^{II} und R^{V} jeweils beide für Methyl, Ethyl, Isopropyl oder tert.-Butyl stehen und R^{I}, R^{III}, R^{IV} und R^{VI} für Wasserstoff stehen.

Insbesondere steht Y für eine Gruppe der Formel (VII.b), worin R^{I}, R^{II}, R^{III}, R^{IV}, R^{V} und R^{VI} alle für Wasserstoff stehen.

In einer ersten bevorzugten Ausführungsform weisen die als Hydroformylierungskatalysatoren eingesetzten Rhodiumkomplexe wenigstens ein Chelatphosphoramidit als Liganden auf. Besonders bevorzugt umfasst der eingesetzte Hydroformylierungskatalysator wenigstens eine Phosphorchelatverbindung der allgemeinen Formel (VI.1) 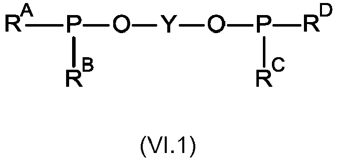 worin
- Y: die zuvor angegebene Bedeutung besitzt,
die Reste R^{A}, R^{B}, R^{C} und R^{D} unabhängig voneinander ausgewählt sind unter über das pyrrolische Stickstoffatom an das Phosphoratom gebundene Pyrrolgruppen.

Bevorzugt sind in den Verbindungen der allgemeinen Formel (VI.1) die Reste R^{A} und R^{B} und die Reste R^{C} und R^{D} nicht miteinander verbunden. Die Bedeutung des Begriffs Pyrrolgruppe entspricht dabei der zuvor gegebenen Definition.

Besonders bevorzugt umfasst der eingesetzte Hydroformylierungskatalysator wenigstens eine Phosphorchelatverbindung der allgemeinen Formel (VI.1) 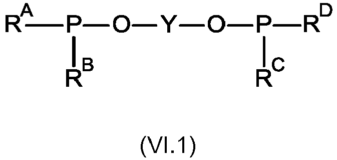 worin
- Y: die zuvor angegebene Bedeutung besitzt,
die Reste R^{A}, R^{B}, R^{C} und R^{D} unabhängig voneinander ausgewählt sind unter Gruppen der Formeln VIII.a bis VIII.k 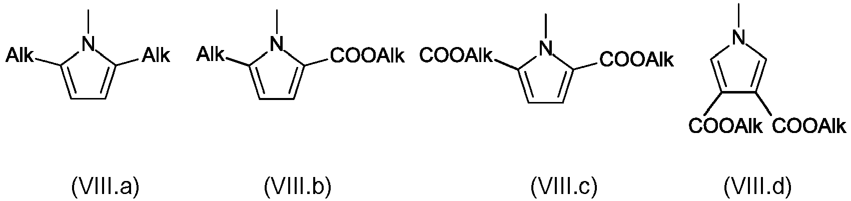 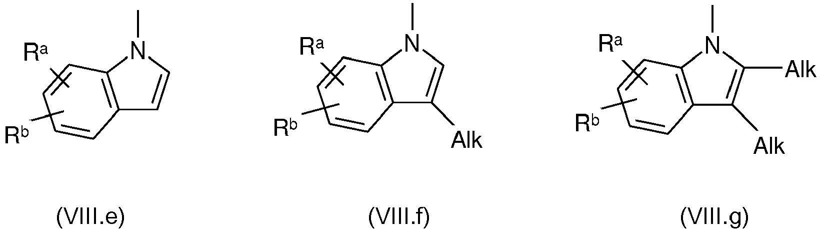 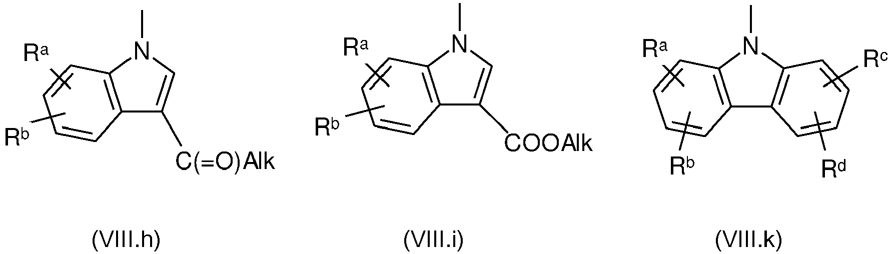 worin
Alk eine C₁-C₄-Alkylgruppe ist und
R^{a}, R^{b}, R^{c} und R^{d} unabhängig voneinander für Wasserstoff, C₁-C₄-Alkyl, C₁-C₄-Alkoxy, Formyl, Acyl, Halogen, C₁-C₄-Alkoxycarbonyl oder Carboxyl stehen.

Bevorzugt haben in den Verbindungen der Formel (VI.1) die Reste R^{A}, R^{B}, R^{C} und R^{D} alle die gleiche Bedeutung.

Bevorzugte Alkylreste R^{a} bis R^{d} in den Verbindungen der Formel (VI.1) sind Methyl, Ethyl, n-Propyl, Isopropyl, n-Butyl, tert.-Butyl und Trifluormethyl.

In einer speziellen Ausführungsform stehen in den Verbindungen der Formel (VI.1) die Reste R^{A}, R^{B}, R^{C} und R^{D} für die 3-Methylindolylgruppe (Skatolylgruppe).

Insbesondere steht in den Verbindungen (VI.1) die Gruppe Y für eine Gruppe der Formel (VII.a), worin R^{II} und R^{V} jeweils beide für Methyl, Ethyl, Isopropyl oder tert.-Butyl stehen und R^{I}, R^{III}, R^{IV} und R^{VI} für Wasserstoff stehen.

In einer zweiten bevorzugten Ausführungsform weisen die als Hydroformylierungskatalysatoren eingesetzten Rhodiumkomplexe wenigstens ein Chelatphosphit als Liganden auf. Besonders bevorzugt umfasst der eingesetzte Hydroformylierungskatalysator wenigstens eine Phosphorchelatverbindung der allgemeinen Formel (VI.2) 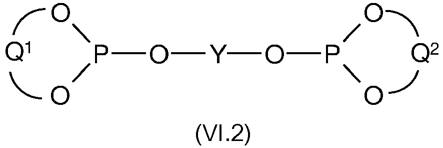 worin
- Y: die zuvor angegebene Bedeutung besitzt,
- Q¹ und Q²: unabhängig voneinander für eine zweiwertige verbrückende Gruppe der allgemeinen Formel (IX) stehen, 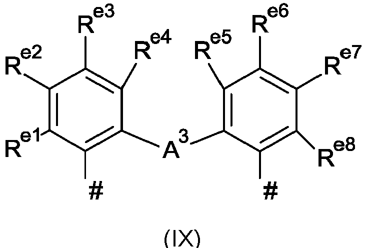
worin
# jeweils für eine Bindungsstelle an die Phosphit-Gruppe steht,
R^{e1}, R^{e2}, R^{e3}, R^{e4}, R^{e5}, R^{e6}, R^{e7} und R^{e8} unabhängig voneinander für Wasserstoff, jeweils unsubstituiertes oder substituiertes Alkyl, Alkoxy, Cycloalkyl, Cycloalkoxy, Heterocycloalkyl, Heterocycloalkoxy, Aryl, Aryloxy, Hetaryl, Hetaryloxy,
   Halogen, Hydroxy, Mercapto, Cyano, Nitro, Formyl, Acyl, Carboxy, Carboxylate, Alkylcarbonyloxy, Carbamoyl, Sulfo, Sulfonat oder NE¹²E¹³ stehen, worin E¹² und E¹³ jeweils gleiche oder verschiedene Reste, ausgewählt unter Wasserstoff, Alkyl, Cycloalkyl, Heterocycloalkyl, Aryl oder Hetaryl bedeuten,
wobei zwei benachbarte Reste R^{e1} bis R^{e8} gemeinsam mit den Kohlenstoffatomen des Benzolkerns, an die sie gebunden sind, auch für ein kondensiertes Ringsystem mit 1, 2 oder 3 weiteren Ringen stehen können,
A³ für eine Einfachbindung, O, S, NR^{a31}, SiR^{a32}R^{a33} oder C₁-C₄-Alkylen steht, das eine Doppelbindung und/oder einen Alkyl-, Cycloalkyl-, Heterocycloalkyl-, Aryl- oder Hetaryl-Substituenten aufweisen kann, oder durch O, S, NR^{a31} oder SiR^{a32}R^{a33} unterbrochen sein kann, wobei R^{a31}, R^{a32} und R^{a33} unabhängig voneinander für Wasserstoff, Alkyl, Cycloalkyl, Heterocycloalkyl, Aryl oder Hetaryl stehen.

Bevorzugt steht in den Verbindungen (VI.2) wenigstens eine der Gruppen Q¹ oder Q² für eine Gruppe der Formel (IX), worin A³ für eine Einfachbindung steht, R^{e1} und R^{e8} unabhängig voneinander für C₁-C₄-Alkyl oder C₁-C₄-Alkoxy stehen und R^{e2}, R^{e3}, R^{e4}, R^{e5}, R^{e6} und R^{e7} für Wasserstoff stehen. Vorzugsweise sind R^{e1} und R^{e8} unabhängig voneinander ausgewählt unter Methyl, Ethyl, Isopropyl, tert.-Butyl und Methoxy.

Bevorzugt steht in den Verbindungen (VI.2) wenigstens eine der Gruppen Q¹ oder Q² für eine Gruppe der Formel (IX), worin A³ für eine Einfachbindung steht, R^{e4} und R^{e5} unabhängig voneinander für C₁-C₄-Alkyl oder C₁-C₄-Alkoxy stehen und R^{e1}, R^{e2}, R^{e3}, R^{e6}, R^{e7} und R^{e8} für Wasserstoff stehen. Vorzugsweise sind R^{e4} und R^{e5} unabhängig voneinander ausgewählt unter Methyl, Ethyl, Isopropyl, tert.-Butyl und Methoxy.

Bevorzugt steht in den Verbindungen (VI.2) wenigstens eine der Gruppen Q¹ oder Q² für eine Gruppe der Formel (IX), worin A³ für eine Einfachbindung steht, und R^{e1}, R^{e2}, R^{e3}, R^{e4}, R^{e5}, R^{e6}, R^{e7} und R^{e8} für Wasserstoff stehen.

Besonders bevorzugt steht in den Verbindungen (VI.2) wenigstens eine der Gruppen Q¹ oder Q² für eine Gruppe der Formel (IX), worin A³ für eine Einfachbindung steht, R^{e1}, R^{e3}, R^{e6} und R^{e8} unabhängig voneinander für C₁-C₄-Alkyl oder C₁-C₄-Alkoxy stehen und R^{e2}, R^{e4}, R^{e5} und R^{e7} für Wasserstoff stehen. Vorzugsweise sind R^{e1}, R^{e3}, R^{e6} und R^{e8} unabhängig voneinander ausgewählt unter Methyl, Ethyl, Isopropyl, tert.-Butyl und Methoxy. Insbesondere stehen R^{e1}, R^{e3}, R^{e6} und R^{e8} alle für Methyl.

Insbesondere steht in den Verbindungen (VI.2) die Gruppe Y für eine Gruppe der Formel (VII.b), worin R^{II} und R^{V} jeweils beide für Methyl, Ethyl, Isopropyl oder tert.-Butyl stehen und R^{I}, R^{III}, R^{IV} und R^{VI} alle für Wasserstoff stehen.

Bevorzugt ist in den Verbindungen (VI.2) die Gruppe Y ausgewählt unter 3,3',5,5'-Tetramethyl-1,1'-biphenyl-2,2'-diyl, 3,3',5,5'-Tetraethyl-1,1'-biphenyl-2,2'-diyl, 3,3',5,5'-Tetra-n-propyl-1,1'-biphenyl-2,2'-diyl, 3,3'-Dimethyl-5,5'-dichlor-1,1'-biphenyl-2,2'-diyl, 3,3'-Diethyl-5,5'-dibrom-1,1'-biphenyl-2,2'-diyl, 3,3'-Dimethyl-5,5'-diiod-1,1'-biphenyl-2,2'-diyl, 3,3'-Dimethyl-5,5'-diethyl-1,1'-biphenyl-2,2'-diyl, 3,3'-Dimethyl-5,5'-di-n-propyl-1,1'-biphenyl-2,2'-diyl, 3,3',5,5'-Tetra-isopropyl-1,1'-biphenyl-2,2'-diyl, 3,3',5,5'-Tetra-n-butyl-1,1'-biphenyl-2,2'-diyl, 3,3',5,5'-Tetra-isobutyl-1,1'-biphenyl-2,2'-diyl, 3,3',5,5'-Tetra-sec-butyl-1,1'-biphenyl-2,2'-diyl, 3,3',5,5'-Tetra-tert-butyl-1,1'-biphenyl-2,2'-diyl, 3,3'-Di-tert-butyl-5,5'-di-n-amyl-1,1'-biphenyl-2,2'-diyl, 3,3',5,5'-Tetrakis(1,1-dimethylpropyl)-1,1'-biphenyl-2,2'-diyl, 3,3'-Di-tert-butyl-5,5'-bis(1,1-dimethylpropyl)-1,1'-biphenyl-2,2'-diyl, 3,3'-Di-tert-butyl-5,5'-di-n-hexyl-1,1'-biphenyl-2,2'-diyl, 3,3'-Di-tert-butyl-5,5'-di-2-hexyl-1,1'-biphenyl-2,2'-diyl, 3,3'-Di-tert-butyl-5,5'-di-3-hexyl-1,1'-biphenyl-2,2'-diyl, 3,3'-Di-tert-butyl-5,5'-di-n-heptyl-1,1'-biphenyl-2,2'-diyl, 3,3'-Di-tert-butyl-5,5'-di-2-heptyl-1,1'-biphenyl-2,2'-diyl, 3,3'-Di-tert-butyl-5,5'-di-3-heptyl-1,1'-biphenyl-2,2'-diyl, 3,3'-Di-tert-butyl-5,5'-di-4-heptyl-1,1'-biphenyl-2,2'-diyl, 3,3'-Di-tert-butyl-5,5'-di-n-octyl-1,1'-biphenyl-2,2'-diyl, 3,3'-Di-tert-butyl-5,5'-di-2-octyl-1,1'-biphenyl-2,2'-diyl, 5,5'-Di-3-octyl-1,1'-biphenyl-2,2'-diyl, 3,3'-Di-tert-butyl-5,5'-di-4-octyl-1,1'-biphenyl-2,2'-diyl, 3,3'-Di-tert-butyl-5,5'-bis(1,1,3,3-tetramethylbutyl)-1,1'-biphenyl-2,2'-diyl, 3,3',5,5'-Tetrakis(1,1,3,3-tetramethylbutyl)-1,1'-biphenyl-2,2'-diyl, 3,3'-di-tert-butyl-5,5',6,6'-Tetramethyl-1,1'-biphenyl-2,2'-diyl, 3,3'-Di-tert-butyl-5,5'-diphenyl-1,1'-biphenyl-2,2'-diyl, 3,3'-Di-tert-butyl-5,5'-bis(2,4,6,-trimethylphenyl)-1,1'-biphenyl-2,2'-diyl, 3,3'-Di-tert-butyl-5,5'-dimethoxy-1,1'-biphenyl-2,2'-diyl, 3,3'-Di-tert-butyl-5,5'-diethoxy-1,1'-biphenyl-2,2'-diyl, 3,3'-Di-tert-butyl-5,5'-di-n-propoxy-1,1'-biphenyl-2,2'-diyl, 3,3'-Di-tert-butyl-5,5'-di-isopropoxy-1,1'-biphenyl-2,2'-diyl, 3,3'-Di-tert-butyl-5,5'-di-n-butoxy-1,1'-biphenyl-2,2'-diyl, 3,3'-Di-tert-butyl-5,5'-di-sec-butoxy-1,1'-biphenyl-2,2'-diyl, 3,3'-Di-tert-butyl-5,5'-di-iso-butoxy-1,1'-biphenyl-2,2'-diyl, 3,3'-Di-tert-butyl-5,5'-di-tert-butoxy-1,1'-biphenyl-2,2'-diyl und 1,1'-Binaphthalinyl-2,2'-diyl.

Besonders bevorzugt steht die Gruppe Y für 3,3',5,5'-Tetramethyl-1,1'-biphenyl-2,2'-diyl.

Besonders bevorzugt umfasst der eingesetzte Hydroformylierungskatalysator wenigstens eine Phosphorchelatverbindung als Liganden, die ausgewählt ist unter den Verbindungen:

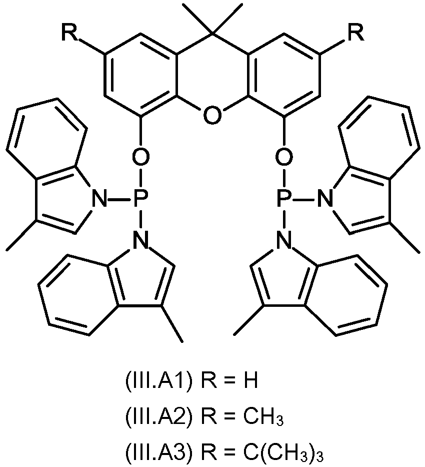

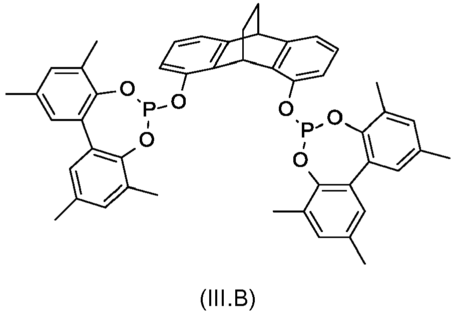

Besonders bevorzugt wird in dem erfindungsgemäßen Verfahren ein Hydroformylierungskatalysator eingesetzt, der [5-Bis(3-methylindol-1-yl)phosphanyloxy-2,7-ditert-butyl-9,9-dimethyl-xanthen-4-yl]oxy-bis(3-methylindol-1-yl)phosphan (tBuSkatOX) als Liganden umfasst.

Des Weiteren besonders bevorzugt wird in dem erfindungsgemäßen Verfahren ein Hydroformylierungskatalysator eingesetzt, der die Verbindung (III.B) (tMe-Rucaphosphit) als Liganden umfasst.

tBuSkatOX und tMe-Rucaphosphit erlauben die Hydroformylierung von Verbindungen der allgemeinen Formel (II) und speziell von β-Farnesen mit hohen Umsätzen, hohen Selektivitäten bezüglich der Hydroformylierung der Vinylgruppe und hohen Ausbeuten an der Verbindung (I).

Zusätzlich zu den zuvor beschriebenen als Liganden eingesetzten Phosphorchelatverbindung als Liganden können die erfindungsgemäß eingesetzten Hydroformylierungskatalysatoren noch wenigstens einen weiteren Liganden aufweisen, der vorzugsweise ausgewählt ist unter Halogeniden, Aminen, Carboxylaten, Acetylacetonat, Aryl- oder Alkylsulfonaten, Hydrid, CO, Olefinen, Dienen, Cycloolefinen, Nitrilen, N-haltigen Heterocyclen, Aromaten und Heteroaromaten, Ethern, PF₃, Phospholen, Phosphabenzolen sowie einzähnigen Phosphin-, Phosphinit-, Phosphonit-, Phosphoramidit- und Phosphitliganden aufweisen. Besonders geeignete weitere Liganden sind Hydrid, Carbonyl und Triphenylphosphin. Der Übergangsmetallkomplexkatalysator kann mehrere verschiedene der genannten Liganden enthalten. Besonders bevorzugt enthält der Übergangsmetallkomplexkatalysator einen Phosphorchelatliganden, Hydrid und Carbonyl oder einen Phosphorchelatliganden und Hydrid oder einen Phosphorchelatliganden und Carbonyl.

Die Menge der Metallkomponenten des Katalysators, bevorzugt Rhodium, beträgt im Allgemeinen 0,1 bis 5000 Gewichts-ppm, jeweils bezogen auf das gesamte flüssige Reaktionsgemisch.

Das Molmengenverhältnis von phosphorhaltigem Ligand zu Metall liegt im Allgemeinen in einem Bereich von etwa 1 : 1 bis 1000 : 1, bevorzugt in einem Bereich von 1 : 1 bis 500 : 1.

Die homogenen Katalysatoren können sowohl direkt in ihrer aktiven Form eingesetzt werden, als auch ausgehend von Übergangsmetallquellen unter Zugabe der entsprechenden Liganden, speziell der oben genannten mehrzähnigen Phosphorchelatverbindungen, erst unter den Reaktionsbedingungen erzeugt werden. Bevorzugt ist ein Verfahren, das dadurch gekennzeichnet ist, dass der Hydroformylierungskatalysator in situ hergestellt wird, wobei man mindestens einen der Liganden, eine Verbindung oder einen Komplex des Metalls und gegebenenfalls ein Aktivierungsmittel in einem inerten Lösungsmittel unter den Hydroformylierungsbedingungen zur Reaktion bringt.

Geeignete Übergangsmetallquellen sind ganz allgemein Übergangsmetalle, Übergangsmetallverbindungen und Übergangsmetallkomplexe, woraus in der Reaktionszone unter den Hydroformylierungsbedingungen der Hydroformylierungskatalysator in situ gebildet wird.

Als Übergangsmetallquelle geeignete Rhodiumverbindungen oder-komplexe sind z. B. Rhodium(II)- und Rhodium(III)-salze, wie Rhodium(II)- bzw. Rhodium(III)-carboxylat, Rhodium(II)- und Rhodium(III)-acetat, etc. Weiterhin eignen sich Rhodiumkomplexe, wie Rhodiumbiscarbonylacetylacetonat, Acetylacetonatobisethylenrhodium(I) Acetylacetonatocyclooctadienylrhodium(I), Acetylacetonatonorbornadienylrhodium(I), Acetylacetonatocarbonyltriphenylphosphinrhodium(I), etc.

Die Hydroformylierungsreaktion kann kontinuierlich, semikontinuierlich oder diskontinuierlich erfolgen.

Die Hydroformylierung erfolgt in einer Reaktionszone, die einen oder mehrere, gleiche oder verschiedene Reaktoren umfassen kann. Im einfachsten Fall wird die Reaktionszone von einem einzelnen Reaktor gebildet. Die Reaktoren können jeweils gleiche oder verschiedene Vermischungscharakteristiken aufweisen. Die Reaktoren können gewünschtenfalls durch Einbauten ein- oder mehrfach unterteilt sein. Bilden zwei oder mehrere Reaktoren eine Zone, so können diese untereinander beliebig verschaltet sein, z. B. parallel oder in Reihe. Als Reaktoren können prinzipiell sämtliche für Hydroformylierungsreaktionen geeignete Reaktortypen verwendet werden, beispielsweise Rührreaktoren, Blasensäulenreaktoren, wie sie z. B. in US-A 4,778,929 beschrieben sind, Umlaufreaktoren, wie sie z. B. Gegenstand von EP-A 1 114 017 sind, Rohrreaktoren, wobei die einzelnen Reaktoren eine Reihe unterschiedliche Mischungscharakteristiken haben können, wie z. B. in EP-A 423 769 beschrieben; des Weiteren können kompartimentierte Reaktoren verwendet werden, wie sie z. B. Gegenstand von EP-A 1 231 198 oder von US-A 5,728,893 sind. Geeignete Reaktoren für die kontinuierliche Umsetzung sind im Übrigen dem Fachmann bekannt und werden z. B. in Ullmanns Enzyklopädie der technischen Chemie, Bd. 1, 3. Aufl., 1951, S. 743 ff. beschrieben.

Geeignete druckfeste Reaktoren sind dem Fachmann ebenfalls bekannt und werden z. B. in Ullmanns Enzyklopädie der technischen Chemie, Bd. 1, 3. Auflage, 1951, S. 769 ff. beschrieben. Im Allgemeinen wird für das erfindungsgemäße Verfahren ein Autoklav verwendet, der gewünschtenfalls mit einer Rührvorrichtung und einer Innenauskleidung versehen sein kann.

Die Zusammensetzung des im erfindungsgemäßen Verfahren eingesetzten Synthesegases aus Kohlenmonoxid und Wasserstoff kann in weiten Bereichen variieren. Das molare Verhältnis von Kohlenmonoxid und Wasserstoff beträgt in der Regel etwa 5 : 95 bis 70 : 30, bevorzugt etwa 40 : 60 bis 60 : 40. Insbesondere bevorzugt wird ein molares Verhältnis von Kohlenmonoxid und Wasserstoff im Bereich von etwa 1 : 1 eingesetzt.

Die Temperatur bei der Hydroformylierungsreaktion liegt im Allgemeinen in einem Bereich von etwa 20 bis 180 °C, bevorzugt etwa 50 bis 150 °C, besonders bevorzugt 60 bis 100 °C. Die Reaktion wird in der Regel bei dem Partialdruck des Reaktionsgases bei der gewählten Reaktionstemperatur durchgeführt. Im Allgemeinen liegt der Druck in einem Bereich von etwa 1 bis 700 bar, bevorzugt 1 bis 100 bar, insbesondere 5 bis 60 bar. Der Reaktionsdruck kann in Abhängigkeit von der Aktivität des eingesetzten erfindungsgemäßen Hydroformylierungskatalysators variiert werden. Im Allgemeinen erlauben die erfindungsgemäßen Katalysatoren auf Basis von phosphorhaltigen Verbindungen eine Umsetzung in einem Bereich niedriger Drücke, wie etwa im Bereich von 1 bis 100 bar.

Die Hydroformylierung kann in einem geeigneten, unter den jeweiligen Reaktionsbedingungen inerten Lösungsmittel durchgeführt werden. Geeignete Lösungsmittel sind z. B. die bei der Hydroformylierung gebildeten Aldehyde und höher siedende Reaktionskomponenten, z. B. die Produkte der Aldolkondensation. Weiterhin geeignet sind Aromaten, wie Toluol und Xylole, Kohlenwasserstoffe oder Gemische von Kohlenwasserstoffen, Ester aliphatischer Carbonsäuren mit Alkanolen, beispielsweise Texanol®, und Ester aromatischer Carbonsäuren , z. B. C₈-C₁₃-Dialkylphthalate.

Die erfindungsgemäßen Hydroformylierungskatalysatoren lassen sich nach üblichen, dem Fachmann bekannten Verfahren vom Austrag der Hydroformylierungsreaktion abtrennen und können im Allgemeinen erneut für die Hydroformylierung eingesetzt werden.

Vorteilhafterweise zeigen die erfindungsgemäßen Katalysatoren eine hohe Aktivität, so dass in der Regel die entsprechenden Aldehyde in guten Ausbeuten erhalten werden. Die Hydroformylierungsaktivität der erfindungsgemäßen Katalysatoren ist überraschenderweise in der Regel höher als die Isomerisierungsaktivität bezüglich der Doppelbindungen, welche nicht durch Hydroformylierung umgesetzt werden.

Das erhaltene Reaktionsgemisch kann einer Aufarbeitung nach üblichen, dem Fachmann bekannten Verfahren unterzogen werden. Dazu kann das bei der Hydroformylierung erhaltene Reaktionsgemisch wenigstens einem Aufarbeitungsschritt zur Abtrennung wenigstens einer der folgenden Komponenten:
- Hydroformylierungskatalysator,
- nicht umgesetzten Verbindungen der Formel (I),
- von den Verbindungen der Formel (11.1) verschiedenen Reaktionsprodukten,
- Lösungsmittel,
unterzogen werden.

### Hydrierung (Schritt b)

In Schritt b) des erfindungsgemäßen Verfahrens wird wenigstens ein Aldehyd der allgemeinen Formel (I) einer Hydrierung unter Erhalt wenigstens eine Verbindung der allgemeinen Formel (III) R¹-C(=CH₂)-(CH₂)₂-CH₂-OH unterzogen. In einer speziellen Ausführungsform wird (7E)-8,12-Dimethyl-4-methylen-trideca-7,11-dienal (I.1) einer Hydrierung unter Erhalt einer Verbindung der Formel (III.1) unterzogen. Im Rahmen der erfindungsgemäßen Herstellung von (-)-Ambrox wird dieser Reaktionsschritt als Schritt b1) bezeichnet. Soweit im Folgenden nicht anderes angegeben ist, gelten die Ausführungen zu Schritt b) analog für den Schritt b1).

Die erfindungsgemäß zur Hydrierung eingesetzten Katalysatoren ermöglichen eine gute Selektivität bezüglich der Hydrierung der endständigen Aldehydgruppe unter Erhalt des entsprechenden Alkohols. Die Hydrierung und die Isomerisierung der in den Verbindungen (I) bzw. (I.1) enthaltenen Doppelbindungen kann im Wesentlichen vermieden werden.

Bevorzugt wird die Hydrierung in Schritt b) bzw. b1) in Gegenwart eines im Reaktionsgemisch löslichen Übergangsmetall-Katalysators durchgeführt.

Bevorzugte Übergangsmetall-Verbindungen sind solche der Metalle der VIII. Nebengruppe des Periodensystems der Elemente, insbesondere Ru, Rh, Pd, Ir und Pt. Besonders bevorzugt ist Ru.

Geeignet sind beispielsweise Katalysatoren und Katalysatorvorstufen, aus denen unter den Hydrierungsbedingungen katalytisch aktive Spezies der allgemeinen Formel [H_{g}Me_{d}(CO)ₑG_{f}] gebildet werden, worin Me für ein Metall der VIII. Nebengruppe, G für einen zwei- oder mehrzähnigen Phosphor-, Arsen-oder Antimon-haltigen Liganden und d, e, f, g für ganze Zahlen, abhängig von der Wertigkeit und Art des Metalls sowie der Bindigkeit des Liganden G, stehen. Vorzugsweise stehen e und f unabhängig voneinander mindestens für einen Wert von 1, wie z. B. 1, 2 oder 3. Die Summe aus e und f steht bevorzugt für einen Wert von 2 bis 5. Geeignete Liganden G sind ausgewählt unter zwei- und mehrzähnigen Phosphin-, Phosphinit-, Phosphonit-, Phosphoramidit- und Phosphitliganden. Geeignete Katalysatoren können zusätzlich wenigstens einen weiteren Liganden aufweisen, der vorzugsweise ausgewählt ist unter Triarylphosphinen, Triarylphosphiten, Triarylphosphiniten, Triarylphosphoniten, PF₃, Phospholen, Phosphabenzolen, Trialkylphosphinen, Phosphametallocenen, Halogeniden, Aminen, Carboxylaten, Acetylacetonat, Aryl- oder Alkylsulfonaten, Olefinen, Dienen, Cycloolefinen, Ethern, Nitrilen, nichtaromatischen N-haltigen Heterocyclen, Aromaten und Heteroaromaten. Ein besonders bevorzugter weiterer Ligand ist Triphenylphosphin (TPP).

In einer speziellen Ausführungsform wird zur Hydrierung ein Katalysator eingesetzt, der wenigstens eine Verbindung der allgemeinen Formel (X) 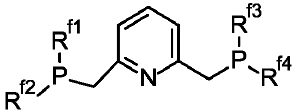 als Liganden aufweist, worin
R^{f1}, R^{f2}, R^{f3} und R^{f4}, unabhängig voneinander ausgewählt sind unter C₁-C₆-Alkyl.

Bevorzugt haben R^{f1}, R^{f2}, R^{f3} und R^{f4} dieselbe Bedeutung.

Ein bevorzugter Ligand ist Di-tert-butyl-[[6-(di-tert-butylphosphanylmethyl)-2-pyridyl]-methyl]phosphan:

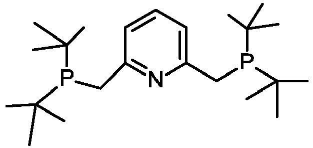

Die Hydrierungskatalysatoren können in situ, in dem für die Hydrierungsreaktion eingesetzten Reaktor, hergestellt werden. Gewünschtenfalls können die erfindungsgemäß in Schritt b) bzw. b1) eingesetzten Katalysatoren auch separat hergestellt und nach üblichen Verfahren isoliert werden. Zur in situ-Herstellung der Katalysatoren kann man eine Verbindung oder einen Komplex eines Metalls der VIII. Nebengruppe, gewünschtenfalls einen oder mehrere Liganden und gegebenenfalls ein Aktivierungsmittel in einem inerten Lösungsmittel unter den Hydrierungsbedingungen umsetzen.

Geeignete Verbindungen der genannten Übergangsmetalle sind insbesondere solche, die im gewählten Reaktionsmedium löslich sind, wie beispielsweise Salze oder Komplexverbindungen mit geeigneten Liganden, wie z. B. Carbonyl, Acetylacetonat, Hydroxy, Cyclooctadien, Norbornadien, Cycloocten, Methoxy, Acetyl oder sonstige aliphatische oder aromatische Carboxylate. Im Rahmen des erfindungsgemäßen Verfahrens bevorzugte Übergangsmetallverbindungen sind Ru(II), Ru(III), Ru(IV)-, Ru(0)-, Rh(I)-, Rh(III)-, Rh(0)-, Ir(I)-, Ir(III), Ir(IV)-, Ir(0)-Verbindungen, Pd(II)-, Pd(IV)-, Pd(0)-, Pt(II)-, Pt(IV)- sowie Pt(0)-Verbindungen.

Geeignete Rutheniumsalze sind beispielsweise Ruthenium(III)chlorid, Ruthenium(IV)-, Ruthenium(VI)oxid oder Ruthenium(VIII)oxid, Alkalisalze der Rutheniumsauerstoffsäuren, wie K₂RuO₄ oder KRuO₄, oder Komplexverbindungen des Rutheniums. Dazu zählen die Metallcarbonyle, wie Trisrutheniumdodecacarbonyl oder Hexarutheniumoctadecacarbonyl, oder Mischformen, in denen CO teilweise durch Liganden der Formel PR₃ ersetzt sind, wie Ru(CO)₃(TPP)₂.

Bevorzugt sind solche Übergangsmetallverbindungen, die bereits mindestens einen CO-Liganden aufweisen. Daneben lassen sich auch Übergangsmetall-Verbindungen, die keinen CO-Liganden aufweisen, als Ausgangsverbindung zur Herstellung der erfindungsgemäß einzusetzenden Katalysatoren verwenden. Diese können dann in einer Präformierung mit CO bzw. unter den Hydrierbedingungen unter Zusatz von Kohlenmonoxid in die gewünschten Katalysatoren überführt werden.

Insbesondere erfolgt die Hydrierung in Schritt b) bzw. b1) in Gegenwart von [HRuCl(CO)(TPP)₃ als Hydrierkatalysator.

Die genannten Übergangsmetallverbindungen setzt man üblicherweise in einer Menge von etwa 0,01 bis etwa 1 Mol-%, bevorzugt von etwa 0,05 bis etwa 0,5 Mol-%, insbesondere von etwa 0,02 bis etwa 0,2 Mol-% (bezogen auf die enthaltenen Übergangsmetallatome) im Verhältnis zur Menge an zu hydrierendem Substrat ein.

Bei unter kontinuierlichen Bedingungen durchgeführten Umsetzungen wählt man das Verhältnis von eingesetzter Menge an Übergangsmetallverbindung als Vorläufer des erfindungsgemäßen homogenen Katalysators zur Menge an zu hydrierendem Substrat vorteilhaft so, dass die Katalysatorkonzentration im Reaktionsgemisch im Bereich von etwa 10 ppm bis 100000 ppm, besonders bevorzugt 50 ppm bis 50000 ppm, insbesondere im Bereich von 100 ppm bis 10000 ppm, liegt.

Die Hydrierung in Schritt b) bzw. b1) erfolgt vorteilhaft bei einem Druck von etwa 1 bis etwa 300 bar (0,1-30 MPa), bevorzugt von etwa 2 bis etwa 200 bar (0,2-20 MPa), insbesondere bei etwa 3 bis etwa 100 bar (0,3-10 MPa) speziell von etwa 5 bis etwa 50 bar (0,5 -5 MPa).

Die Hydrierung in Schritt b) bzw. b1) erfolgt vorteilhaft bei einer Temperatur von etwa 0 bis 320 °C, bevorzugt von 20 bis 300 °C, insbesondere von 50 bis 280 °C.

Geeignete Lösemittel sind beispielsweise Ether, Tetrahydrofuran, Toluol, Chlorbenzol, Octadecanol, Biphenylether, Texanol, Marlotherm, Oxoöl 9N (Hydroformylierungsprodukte aus isomeren Octenen, BASF SE).

Üblicherweise ist die Hydrierung nach etwa 1 bis etwa 150 h, oft nach etwa 2 bis etwa 48 h, abgeschlossen.

Das erhaltene Reaktionsprodukt kann durch dem Fachmann an sich bekannte Verfahren, wie z. B. durch Destillation, aus dem Reaktionsgemisch entfernt werden und der zurückbleibende Katalysator kann, gegebenenfalls nach abermaliger Präformierung, im Rahmen weiterer Umsetzungen genutzt werden.

Die Hydrierung in Schritt b) bzw. b1) kann diskontinuierlich bzw. oder kontinuierlich betrieben werden und eignet sich insbesondere für Umsetzungen in technischem Maßstab.

### Isomerisierung (Schritt c)

In Schritt c) des erfindungsgemäßen Verfahrens wird wenigstens ein Alkohol der allgemeinen Formel (III) einer Isomerisierung unter Erhalt wenigstens einer Verbindung der allgemeinen Formel (IV) unterzogen:

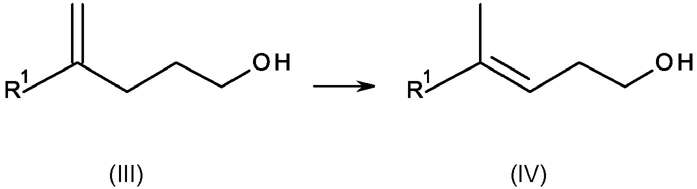

In einer speziellen Ausführungsform wird (7E)-8,12-Dimethyl-4-methylen-trideca-7,11-dienol (III.1) einer Isomerisierung unter Erhalt von (3*E*,7*E*)-Homofarnesol (IV.1) unterzogen. Im Rahmen der erfindungsgemäßen Herstellung von (-)-Ambrox wird dieser Reaktionsschritt als Schritt c1) bezeichnet. Soweit im Folgenden nicht anderes angegeben ist, gelten die Ausführungen zu Schritt c) analog für den Schritt c1).

Ein geeignetes Verfahren zur Isomerisierung von Alkohol der allgemeinen Formel (III) unter Erhalt von Isomeren der allgemeinen Formel (IV) ist in der WO 2014/114615 beschrieben, auf die hier in vollem Umfang Bezug genommen wird.

Bevorzugt erfolgt die Isomerisierung in Schritt c) bzw. c1) in Gegenwart eines Katalysators der Formel

[Ru(dienyl)₂H]A

worin
- dienyl: für eine C₅-C₂₂-Kohlenwasserstoffgruppe steht, die eine Kohlenstoff-Kohlenstoff-Doppelbindung und eine Gruppe C=C-C- aufweist, und
- A: für ein schwach koordinierendes oder nicht koordinierendes Anion steht.

In einer bevorzugten Ausführungsform der Erfindung steht die Gruppe A für ein schwach oder nicht koordinierendes Monoanion. Bevorzugt steht A für NO₃⁻, HSO₄-, BF₄⁻, PF₆⁻, SbF₆⁻, AsF₆⁻, F- oder R^{X}SO₃⁻, wobei R^{X} für ein Fluoratom oder eine C₁₋₈-Alkylgruppe oder eine C₁₋₈-Fluoralkylgruppe steht. Fluoralkylgruppe bezeichnet dabei eine teilweise oder vollständig fluorierte Alkylgruppe, wie CF₃.

In einer bevorzugten Ausführungsform der Erfindung, steht die Gruppe A für ein Monoanion.

Insbesondere steht die Gruppe A für BF₄⁻, PF₆⁻, SbF₆⁻, AsF₆⁻, F⁻ oder R^{X}SO₃⁻, wobei R^{X} für ein Fluoratom oder CH₃, CF₃, Phenyl oder p-Tolyl steht.

In einer speziellen Ausführungsform steht die Gruppe A für BF₄⁻.

In einer bevorzugten Ausführungsform steht "dienyl" für ein deprotoniertes Dien, wobei Dien für eine C₅-C₂₂-Kohlenwasserstoffgruppe steht, die zwei Kohlenstoff-KohlenstoffDoppelbindungen aufweist und nicht aromatisch ist. Daher umfasst "dienyl" eine Kohlenstoff-Kohlenstoff-Doppelbindung und eine Gruppe C=C-C⁻.

In einer bevorzugten Ausführungsform der Erfindung ist "dienyl" und das Ausgangsdien eine lineare, verzweigte oder cyclische Gruppe.

Bevorzugt steht "dienyl" für ein deprotoniertes Dien, wobei das Dien für eine C₅-C₁₂-Kohlenwasserstoffgruppe steht.

Geeignete "dienyle" sind Cyclooctadienyle, wie, 2,4-Dimethylpentadienyl, 2,3,4-Trimethylpenta-1,3-dienyl, 2,7-Dimethyloctadienyl oder Cycloheptadienyl. Entsprechend ist das Dien, von dem sich der Dienylrest ableitet, ausgewählt unter 1,3-, 1,4- oder 1,5-Cyclooctadien, Norbornadien, 2,4-dimethyl-1,3-pentadien, 2,3,4-Trimethylpenta-1,3-dien, 2,7-Dimethyl-2,6-octadien oder Cyclohepta-1,4-dien.

In einer bevorzugten Ausführungsform ist der Komplex [Ru(dienyl)₂H]A ausgewählt unter [Ru(cycooctadienyl)2H]A, [Ru(cycloheptadienyl)2H]A, [Ru(2,3,4-trimethylpenta-1,3-dienyl)2H]A oder [Ru(2,4-dimethyl-pentadienyl)2H]A.

Geeignete Komplexe [Ru(dienyl)₂H]A sind bekannt oder können nach bekannten Verfahren hergestellt werden, wie sie z. B. in FR 2887253, EP 1283843, in Salzer et al., OM 2000, (19),5471, in Cox and Roulet, Chem. Commun., 1988, 951 oder in F. Bouachir et al, Nouv. J. Chim., 1987, 11, 527 beschrieben sind.

Die Einsatzmenge des Komplexes ist prinzipiell nicht kritisch. Er kann beispielsweise in einer Menge von 0,01 bis 15 Mol-%, bevorzugt von 0,2 bis 2 Mol-%, bezogen auf die zu isomerisierende Verbindung eingesetzt werden.

Die Isomerisierung in Schritt c) bzw. c1) kann in Gegenwart eines unter den Reaktionsbedingungen inerten Gases durchgeführt werden. Geeignete inerte Gase sind Stickstoff, Argon und Mischungen davon.

Die Reaktion kann in Gegenwart oder Abwesenheit eines zugesetzten Lösungsmittels erfolgen. Geeignete Lösungsmittel sind aromatische Kohlenwasserstoffe, wie Benzol, Toluol oder Xylol, C₃-C₉-Ester, wie AcOEt, chlorierte Lösungsmittel, wie Methylenchlorid, Chloroform oder Dichlorethan, C₂-C₉-Ether, wie Tetrahydrofuran, Methyltetrahydrofuran, Dimethoxyethane, Diethylether, C₂-C₉-Alkohole, wie Methanol, Ethanol, sowie Mischungen der zuvor genannten Lösungsmittel.

Vorzugsweise liegt die Temperatur bei der Isomerisierung in Schritt b) bzw. b1) in einem Bereich von 10 °C bis 95 °C, bevorzugter von 20 °C bis 60 °C.

### Cyclisierung (Schritt d1)

Nach der speziellen Ausführungsform des erfindungsgemäßen Verfahrens zur Herstellung von (-)-Ambrox wird im abschließenden Schritt d1) (3*E*,7*E*)-Homofarnesol (IV.1) einer Cyclisierung unter Erhalt von (-)-Ambrox (V) unterzogen.

Wie eingangs erwähnt, ist die Cyclisierung des (3*E*,7*E*)-Homofarnesols zum Ambrox bekannt, wobei sowohl enzymatische als auch chemische Cyclisierungen beschrieben sind. Diesbezüglich wird auf die Offenbarung der folgenden Dokumente Bezug genommen:
P. F. Vlad et al. Khimiya Geterotsiklicheskikh Soedinenii, Engl. Transl. 1991, 746 beschreiben Cyclisierungsreaktionen unter Verwendung einer Supersäure (Fluorsulfonsäure in 2-Nitropropan). Ein weiteres geeignetes Verfahren umfasst die enantioselektive Polyencyclisierung von Homofarnesyltriethylsilylether in Gegenwart von O-(o-Fluorbenzyl)-binol und SnCl₄, wie von H. Yamamoto et al. J. Am. Chem. Soc. 2002, 3647 beschrieben.

Bevorzugt ist die biokatalytische Cyclisierung, wie sie in der WO 2010/139719 beschrieben ist. Danach erfolgt die Cyclisierung in Schritt d1) in Gegenwart eines Polypeptids mit der Aktivität einer Homofarnesol-Ambrox-Cyclase als Enzym.

Zur Herstellung von (-)-Ambrox nach dieser Variante wird Homofarnesol mit der Homofarnesol-Ambroxan-Cyclase in Kontakt gebracht und/oder inkubiert und anschließend das gebildete Ambrox isoliert.

In einer Ausführung von Schritt d1) wird Homofarnesol mit der Homofarnesol-Ambroxan-Cyclase in einem Medium in Kontakt gebracht und/oder so inkubiert, dass eine Umsetzung von Homofarnesol zu Ambrox in Gegenwart der Cyclase erfolgt. Bevorzugt handelt es sich bei dem Medium um ein wässriges Reaktionsmedium. Bei den wässrigen Reaktionsmedien handelt es sich vorzugsweise um gepufferte Lösungen, die in der Regel einen pH-Wert von vorzugsweise 5 bis 8 aufweisen. Als Puffer kann ein Citrat-, Phosphat-, TRIS- (Tris(hydroxymethyl)-aminomethan) oder MES-(2-(N-Morpholino)ethansulfonsäure)-Puffer verwendet werden. Ferner kann das Reaktionsmedium noch weitere Zusätze enthalten, wie z. B. Detergentien (beispielsweise Taurodeoxycholat).

Das Homofarnesol wird vorzugsweise in einer Konzentration von 5 bis 100 mM, besonders bevorzugt von 15 bis 25mM, in der enzymatischen Umsetzung eingesetzt. Diese kann kontinuierlich oder diskontinuierlich durchgeführt werden.

Die enzymatische Cyclisierung in Schritt d1) erfolgt zweckmäßig bei einer Reaktionstemperatur unterhalb der Desaktivierungstemperatur der eingesetzten Cyclase. Bevorzugt liegt die Reaktionstemperatur in Schritt d1) in einem Bereich von - 10 bis 100 °C, besonders bevorzugt von 0 bis 80 °C, insbesondere von 15 bis 60 °C und speziell von 20 bis 40 °C.

Das Reaktionsprodukt Ambrox kann mit organischen Lösungsmitteln extrahiert werden und gegebenenfalls zur Aufreinigung destilliert werden. Geeignete Lösungsmittel werden im Folgenden genannt.

Neben einphasigen wässrigen Systemen können auch zweiphasige Systeme eingesetzt werden. In dieser Variante gelingt es, das gebildete Ambrox in der nichtwässrigen Phase anzureichern, um es zu isolieren. Die zweite Phase ist dabei vorzugsweise ausgewählt unter ionischen Flüssigkeiten und organischen nicht-wassermischbaren Lösungsmitteln. Nach der Reaktion ist Ambrox in der organische Phase leicht von der wässrigen Phase, die den Biokatalysator enthält, abtrennbar. Unter nichtwässrigen Reaktionsmedien werden Reaktionsmedien verstanden, die weniger als 1 Gew.-%, vorzugsweise weniger als 0,5 Gew.-% Wasser, bezogen auf das Gesamtgewicht des flüssigen Reaktionsmediums, enthalten. Insbesondere kann die Umsetzung in einem organischen Lösungsmittel durchgeführt werden. Geeignete organische Lösungsmittel sind beispielsweise aliphatische Kohlenwasserstoffe, vorzugsweise mit 5 bis 8 Kohlenstoffatomen, wie Pentan, Cyclopentan, Hexan, Cyclohexan, Heptan, Octan oder Cyclooctan, halogenierte aliphatische Kohlenwasserstoffe, vorzugsweise mit einem oder zwei Kohlenstoffatomen, wie Dichlormethan, Chloroform, Tetrachlorkohlenstoff, Dichlorethan oder Tetrachlorethan, aromatische Kohlenwasserstoffe, wie Benzol, Toluol, die Xylole, Chlorbenzol oder Dichlorbenzol, aliphatische acyclische und cyclische Ether oder Alkohole, vorzugsweise mit 4 bis 8 Kohlenstoffatomen, wie Ethanol, Isopropanol, Diethylether, Methyl-tert-butylether, Ethyl-tert-butylether, Dipropylether, Diisopropylether, Dibutylether, Tetrahydrofuran oder Ester wie Ethylacetat oder n-Butylacetat oder Ketone wie Methylisobutylketon oder Dioxan oder Gemische davon. Besonders bevorzugt werden die vorgenannten Heptan, Methyl-tert-butylether, Diisopropylether, Tetrahydrofuran, Ethylacetat verwendet.

Geeignete Enzyme sind in der WO 2010/139719 beschrieben, worauf hier in vollem Umfang Bezug genommen wird. Danach ist das Enzym ein Polypeptid, welches kodiert wird von einem Nukleinsäuremolekül, umfassend mindestens ein Nukleinsäuremolekül, ausgewählt aus:
a) Nukleinsäuremolekül, das ein Polypeptid kodiert, umfassend die SEQ ID NO 2, 5, 6, 7, 8, 9, 10 oder 11; b) Nukleinsäuremolekül, das zumindest ein Polynukleotid der Sequenz gezeigt in SEQ ID NO 1 umfasst; c) Nukleinsäuremolekül, das ein Polypeptid kodiert, dessen Sequenz eine Identität von mindestens 46 % zu den Sequenzen SEQ ID NO 2, 5, 6, 7, 8, 9, 10 oder 11 aufweist; d) Nukleinsäuremolekül nach (a) bis (c), das für ein funktional äquivalentes Polypeptid oder ein Fragment der Sequenz gemäß SEQ ID NO 2, 5, 6, 7, 8, 9, 10 oder 11 steht; e) Nukleinsäuremolekül, kodierend für ein funktional äquivalentes Polypeptid mit der Aktivität einer Homofarnesol-Ambroxan-Cyclase, das man erhält, indem man ein Nukleinsäuremolekül aus einer cDNA Bank oder aus genomischer DNA mittels der Primer gemäß Sequenz Nr. 3 und 4 amplifiziert, oder das Nukleinsäuremolekül durch de novo-Synthese chemisch synthetisiert; f) Nukleinsäuremolekül, kodierend für ein funktional äquivalentes Polypeptid mit der Aktivität einer Homofarnesol-Ambroxan-Cyclase, das unter stringenten Bedingungen mit einem Nukleinsäuremolekül gemäß (a) bis (c) hybridisiert; g) Nukleinsäuremolekül, kodierend für ein funktional äquivalentes Polypeptid mit der Aktivität einer Homofarnesol-Ambroxan-Cyclase, das aus einer DNA-Bank unter Verwendung eines Nukleinsäuremoleküls gemäß (a) bis (c) oder deren Teilfragmente von mindestens 15 nt, vorzugsweise 20 nt, 30 nt, 50 nt, 100 nt, 200 nt oder 500 nt als Sonde unter stringenten Hybridisierungsbedingungen isoliert werden kann; und h) Nukleinsäuremolekül, kodierend für ein funktional äquivalentes Polypeptid mit der Aktivität einer Homofarnesol-Ambroxan-Cyclase, wobei die Sequenz des Polypeptids eine Identität von mindestens 46 % zu den Sequenzen SEQ ID NO 2, 5, 6, 7, 8, 9, 10 oder 1 1 aufweist; i) Nukleinsäuremolekül, kodierend für ein funktional äquivalentes Polypeptid mit der Aktivität einer Homofarnesol-Ambroxan-Cyclase, wobei das Polypeptid durch ein Nukleinsäuremolekül, ausgewählt aus der Gruppe der in a) bis h) beschriebenen, kodiert wird und mittels eines monoklonalen Antikörpers isoliert wurde oder isolierbar ist; j) Nukleinsäuremolekül, kodierend für ein funktional äquivalentes Polypeptid mit der Aktivität einer Homofarnesol-Ambroxan-Cyclase, wobei das Polypeptid eine analoge oder ähnliche Bindungsstelle aufweist, wie ein Polypeptid, kodiert durch ein Nukleinsäuremolekül, ausgewählt aus der Gruppe der in a) bis h) beschriebenen.

Trotz einer Vielzahl bereits vorhandener Aromachemikalien (Riech- und Geschmacksstoffe) und Verfahren zu ihrer Herstellung besteht ein ständiger Bedarf an neuen Komponenten, um die Vielzahl der für die äußerst diversen Einsatzbereiche gewünschten Eigenschaften erfüllen zu können sowie an einfachen Syntheserouten, um diese zugängig zu machen. Das erfindungsgemäße Verfahren ermöglicht die effektive Herstellung von Verbindungen der allgemeinen Formel (I) die als interessante Synthesebausteine bei der Bereitstellung von neuen und bereits bekannten Aromachemikalien, wie (-)-Ambrox, dienen können. Nach Hydrierung der Aldehydfunktion können Alkohole erhalten werden, die ihrerseits interessante Synthesebausteine sein können und sich für einen Einsatz als Tensidalkohole eignen. Die Erfindung wird durch die nachstehenden Ausführungsbeispiele näher erläutert.

### Beispiele

### Beispiel 1:

5,1 mg Rhodiumbiscarbonylacetylacetonat und 92,3 mg ^{tBu}SkatOX (Ligand-Metallverhältnis = 5 : 1) wurden in 10 g THF gelöst und mittels Spritze in einen mit CO/H₂ (1 : 1) gespülten 100 ml-Autoklaven gegeben. Anschließend wurde bei 10 bar (1 MPa) CO/H₂ (1 : 1) und 70 °C für 30 min. präformiert. Daraufhin wurde entspannt und 10 g *trans*-β-Farnesen mit einer Spritze zugegeben. Die Hydroformylierung wurde bei 70 °C und 40 bar (4 MPa) CO/H₂ (1 : 1) durchgeführt. Eine Analyse des Reaktionsaustrags nach einer Reaktionszeit von 48 Stunden zeigte einen Umsatz von 92,5 % mit einer Selektivität zum Zielprodukt (7E)-8,12-Dimethyl-4-methylen-trideca-7,11-dienal von 70,4 % und einer Ausbeute an Zielprodukt von 65,1 %.

### Beispiel 2:

5,1 mg Rhodiumbiscarbonylacetylacetonat und 92,3 mg ^{tBu}SkatOX (Ligand-Metallverhältnis = 5 : 1) wurden in 10 g THF gelöst und mittels Spritze in einen mit CO/H₂ (1 : 1) gespülten 100 ml-Autoklaven gegeben. Anschließend wurde bei 10 bar (1 MPa) CO/H₂ (1 : 1) und 70 °C für 30 min. präformiert. Daraufhin wurde entspannt und 10 g *trans*-ß-Farnesen mit einer Spritze zugegeben. Die Hydroformylierung wurde bei 70 °C und 40 bar (4MPa) CO/H₂ (1 : 1) durchgeführt. Eine Analyse des Reaktionsaustrags nach einer Reaktionszeit von 70 Stunden zeigte einen Umsatz von 99,3 % mit einer Selektivität zum Zielprodukt (7E)-8,12-Dimethyl-4-methylen-trideca-7,11-dienal von 68,6 % und einer Ausbeute an Zielprodukt von 68,1 %.

### Beispiel 3:

5,1 mg Rhodiumbiscarbonylacetylacetonat und 44,4 mg tMe-Rucaphosphit (Ligand-Metallverhältnis = 3 : 1) wurden in 10 g THF gelöst und mittels Spritze in einen mit CO/H₂ (1 : 1) gespülten 100 ml-Autoklaven gegeben. Anschließend wurde bei 10 bar (1 MPa) CO/H₂ (1 : 1) und 70 °C für 30 min. präformiert. Daraufhin wurde entspannt und 10 g *trans*-β-Farnesen mit einer Spritze zugegeben. Die Hydroformylierung wurde bei 90 °C und 40 bar CO/H₂ (1 : 1) durchgeführt. Eine Analyse des Reaktionsaustrags nach einer Reaktionszeit von 48 Stunden zeigte einen Umsatz von 87,3 % mit einer Selektivität zum Zielprodukt (7E)-8,12-Dimethyl-4-methylen-trideca-7,11-dienal von 71,9 % und einer Ausbeute an Zielprodukt von 62,7 %.

### Beispiel 4:

### Hydrierung von (7E)-8,12-Dimethyl-4-methylen-trideca-7,11-dienal

(7E)-8,12-Dimethyl-4-methylen-trideca-7,11-dienal wurde in einem Autoklaven als Lösung in THF im Gewichtsverhältnis 1 : 1 zur Hydrierung eingesetzt. Bezogen auf Edukt wurden 0,25 Mol-% [HRuCl(CO)(TPP)₃] und Di-tert-butyl-[[6-(di-tert-butylphosphanylmethyl)-2-pyridyl]methyl]phosphan im Gewichtsverhältnis Chelatligand : Ruthenium von 1 : 1,3 zugegeben. Anschließend wurde auf den Autoklaven Wasserstoff aufgepresst und dieser auf 250 °C erhitzt, wobei ein Druck von 12 bar erreicht wurde. Nach 24 Stunden Reaktionsdauer ließ man abkühlen, entspannte den Autoklaven und analysierte den Reaktionsaustrag mittels NMR-Spektroskopie. Die Ausbeute an Produktalkohol lag bei ca. 80 %. Eine Isomerisierung der Doppelbindungen konnte nicht beobachtet werden.

## Patentansprüche

1. Verfahren zur Herstellung von Verbindungen der allgemeinen Formel (I) und von Folgeprodukten davon, 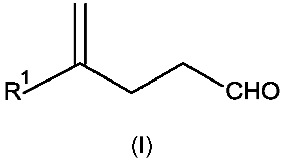 umfassend wenigstens einen Reaktionsschritt, bei dem man wenigstens eine Verbindung der allgemeinen Formel (II) 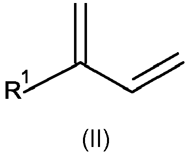 worin
R¹ für jeweils lineares oder verzweigtes C₁-C₂₄-Alkyl oder C₂-C₂₄-Alkenyl mit 1, 2, 3 oder mehr als 3 C-C-Doppelbindungen steht,
einer Hydroformylierung durch Umsetzung mit Kohlenmonoxid und Wasserstoff in Gegenwart eines Hydroformylierungskatalysators unterzieht, der einen Rhodiumkomplex mit wenigstens einer Phosphorchelatverbindung als Liganden umfasst, wobei ein Reaktionsgemisch erhalten wird, das wenigstens 50,1 Gew.-%, bezogen auf das Gesamtgewicht des Reaktionsgemischs, wenigstens einer Verbindung der allgemeinen Formel (I) enthält, wobei der Hydroformylierungskatalysator wenigstens eine Phosphorchelatverbindung der allgemeinen Formel (VI) 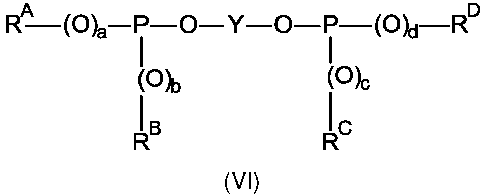 umfasst, worin
R^{A}, R^{B}, R^{C} und R^{D} unabhängig voneinander für Alkyl, Cycloalkyl, Heterocycloalkyl, Aryl oder Hetaryl stehen,
wobei die Alkylreste R^{A}, R^{B}, R^{C} und R^{D} unsubstituiert sind oder 1, 2, 3, 4 oder 5 gleiche oder verschiedene Substituenten tragen, die ausgewählt sind unter Cycloalkyl, Heterocycloalkyl, Aryl, Hetaryl, Alkoxy, Cycloalkoxy, Heterocycloalkoxy, Aryloxy, Hetaryloxy, Hydroxy, Mercapto, Polyalkylenoxid, Polyalkylenimin, COOH, Carboxylat, SO₃H, Sulfonat, NE¹E², NE¹E²E³⁺X⁻, Halogen, Nitro, Formyl, Acyl und Cyano, worin E¹, E² und E³ jeweils gleiche oder verschiedene Reste, ausgewählt unter Wasserstoff, Alkyl, Cycloalkyl, oder Aryl bedeuten und X⁻ für ein Anionäquivalent steht,
und wobei die Cycloalkyl-, Heterocycloalkyl-, Aryl- und Hetarylreste R^{A}, R^{B}, R^{C} und R^{D} jeweils unsubstituiert sind oder 1, 2, 3, 4 oder 5 gleiche oder verschiedene Substituenten tragen, die ausgewählt sind unter Alkyl und den zuvor für die Alkylreste R^{A}, R^{B}, R^{C} und R^{D} genannten Substituenten,
oder
R^{A} und R^{B} und/oder R^{C} und R^{D} zusammen mit dem Phosphoratom und, falls vorhanden, den Sauerstoffatomen, an die sie gebunden sind, für einen 5-bis 8-gliedrigen Heterocyclus stehen, der gegebenenfalls zusätzlich ein-, zwei- oder dreifach mit Cycloalkyl, Heterocycloalkyl, Aryl oder Hetaryl anelliert ist, wobei der Heterocyclus und, falls vorhanden, die anellierten Gruppen unabhängig voneinander unsubstituiert sind oder einen, zwei, drei oder vier gleiche oder verschiedene Substituenten tragen, die ausgewählt sind unter Alkyl, Cycloalkyl, Heterocycloalkyl, Aryl, Hetaryl, Hydroxy, Mercapto, Polyalkylenoxid, Polyalkylenimin, Alkoxy, Halogen, COOH, Carboxylat, SO₃H, Sulfonat, NE⁴E⁵, NE⁴E⁵E⁶⁺X⁻, Nitro, Alkoxycarbonyl, Formyl, Acyl und Cyano, worin E⁴, E⁵ und E⁶ jeweils gleiche oder verschiedene Reste, ausgewählt unter Wasserstoff, Alkyl, Cycloalkyl und Aryl bedeuten und X⁻ für ein Anionäquivalent steht,
a, b, c und d unabhängig voneinander für 0 oder 1 stehen,
Y ausgewählt ist unter Gruppen der Formeln VII.a oder VII.b 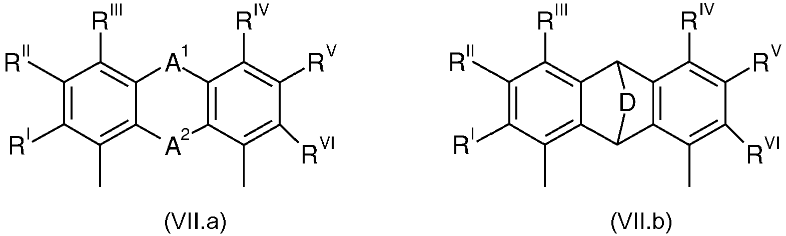
worin
R^{I}, R^{II}, R^{III}, R^{IV}, R^{V} und R^{VI} unabhängig voneinander für Wasserstoff, Alkyl, Cycloalkyl, Heterocycloalkyl, Aryl, Hetaryl, Hydroxy, Mercapto, Polyalkylenoxid, Polyalkylenimin, Alkoxy, Halogen, SO₃H, Sulfonat, NE⁷E⁸, Alkylen-NE⁷E⁸, Trifluormethyl, Nitro, Alkoxycarbonyl, Carboxyl, Formyl, Acyl oder Cyano stehen, worin E⁷ und E⁸ jeweils gleiche oder verschiedene Reste, ausgewählt unter Wasserstoff, Alkyl, Cycloalkyl und Aryl bedeuten,
wobei zwei benachbarte Reste R^{I} bis R^{VI} gemeinsam mit den Kohlenstoffatomen des Benzolkerns, an die sie gebunden sind, auch für ein kondensiertes Ringsystem mit 1, 2 oder 3 weiteren Ringen stehen können,
A¹ und A² unabhängig voneinander für O, S, SiR^{E}R^{F}, NR^{G} oder CR^{H}R^{K} stehen, wobei R^{E}, R^{F}, R^{G}, R^{H} und R^{K} unabhängig voneinander für Wasserstoff, Alkyl, Cycloalkyl, Heterocycloalkyl, Aryl oder Hetaryl stehen,
D für eine zweibindige Brückengruppe der allgemeinen Formel 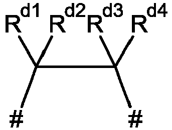
steht, worin
# jeweils für eine Bindungsstelle an das 9,10-Dihydroanthracengerüst steht,
R^{d1}, R^{d2}, R^{d3} und R^{d4} unabhängig voneinander für Wasserstoff, Alkyl, Cycloalkyl, Aryl, Halogen, Trifluormethyl, Carboxyl, Carboxylat oder Cyano stehen,
wobei R^{d1} auch gemeinsam mit R^{d3} für den Bindungsanteil einer Doppelbindung zwischen den beiden Kohlenstoffatomen, an die R^{d1} und R^{d3} gebunden sind, stehen kann, und/oder R^{d2} und R^{d4} gemeinsam mit den Kohlenstoffatomen, an die sie gebunden sind, auch für einen 4- bis 8-gliedrigen Carbo- oder Heterocyclus stehen können, der gegebenenfalls zusätzlich ein-, zwei- oder dreifach mit Cycloalkyl, Heterocycloalkyl, Aryl oder Hetaryl anelliert ist, wobei der Carbo- oder Heterocyclus und, falls vorhanden, die anellierten Gruppen unabhängig voneinander unsubstituiert sind oder je einen, zwei, drei oder vier gleiche oder verschiedene Substituenten tragen, die ausgewählt sind unter Alkyl, Cycloalkyl, Heterocycloalkyl, Aryl, Hetaryl, COOR^{d5}, COO⁻M⁺, SO₃R^{d5}, SO₃⁻M⁺, NE⁹E¹⁰, Alkylen-NE⁹E¹⁰, NE⁹E¹⁰E¹¹⁺ X⁻, Alkylen-NE⁹E¹⁰E¹¹⁺ X⁻, OR^{d6}, SR^{d6}, (CHR^{f}CH₂O)_{y}R^{d6}, (CH₂N(E⁹))_{y}R^{d6}, (CH₂CH₂N(E9))_{y}R^{d6}, Halogen, Trifluormethyl, Nitro, Formyl, Acyl oder Cyano, stehen, worin
R^{d5}, E⁹, E¹⁰ und E¹¹ jeweils gleiche oder verschiedene Reste, ausgewählt unter Wasserstoff, Alkyl, Cycloalkyl oder Aryl bedeuten,
R^{d6} für Wasserstoff, Methyl oder Ethyl steht,
M⁺ für ein Kationäquivalent steht,
X⁻ für ein Anionäquivalent steht, und
y für eine ganze Zahl von 1 bis 120 steht.

2. Verfahren nach Anspruch 1, bei dem man
a) wenigstens eine Verbindung der allgemeinen Formel (II) 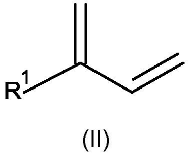 worin
R¹ für jeweils lineares oder verzweigte C₁-C₂₄-Alkyl oder C₂-C₂₄-Alkenyl mit 1, 2, 3 oder mehr als 3 C-C-Doppelbindungen steht,
einer Umsetzung mit Kohlenmonoxid und Wasserstoff in Gegenwart eines Hydroformylierungskatalysators unterzieht, der einen Rhodiumkomplex mit wenigstens einer Phosphorchelatverbindung als Liganden umfasst, wobei ein Reaktionsgemisch erhalten wird, das wenigstens 50,1 Gew.-%, bezogen auf das Gesamtgewicht des Reaktionsgemischs, wenigstens einer Verbindung der allgemeinen Formel (I) enthält,
b) das in Schritt a) erhaltene Reaktionsgemisch oder eine an wenigstens einer Verbindung der allgemeinen Formel (I) angereicherte Fraktion daraus einer Hydrierung unterzieht, wobei ein Reaktionsgemisch erhalten wird, das wenigstens eine Verbindung der allgemeinen Formel (III) 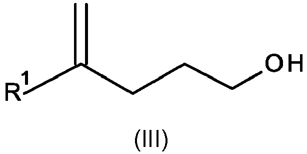 enthält.

3. Verfahren nach einem der Ansprüche 1 oder 2, bei dem man zusätzlich
c) die wenigstens eine Verbindung der allgemeinen Formel (III) einer zumindest teilweisen Isomerisierung unter Erhalt einer Verbindung der allgemeinen Formel (IV) 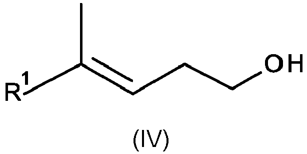 unterzieht.

4. Verfahren nach einem der vorhergehenden Ansprüche, wobei die Verbindungen der Formel (II) nur eine Vinylgruppe im Molekül aufweisen.

5. Verfahren nach einem der vorhergehenden Ansprüche, wobei die Verbindung der allgemeinen Formel (II) ausgewählt ist unter Isopren, β-Myrcen und β-Farnesen.

6. Verfahren nach einem der vorhergehenden Ansprüche, wobei das in der Hydroformylierung (Schritt a)) erhaltene Reaktionsgemisch wenigstens 55 Gew.-%, bevorzugt wenigstens 60 Gew.-%, insbesondere wenigstens 65 Gew.-%, bezogen auf das Gesamtgewicht des Reaktionsgemischs, wenigstens einer Verbindung der allgemeinen Formel (I) enthält.

7. Verfahren nach einem der vorhergehenden Ansprüche, umfassend wenigstens einen Reaktionsschritt, bei dem man β-Farnesen einer Umsetzung mit Kohlenmonoxid und Wasserstoff in Gegenwart eines Hydroformylierungskatalysators unterzieht (= Schritt a)), wobei ein Reaktionsgemisch erhalten wird, das wenigstens 50,1 Gew.-%, bezogen auf das Gesamtgewicht des Reaktionsgemischs, (7E)-8,12-Dimethyl-4-methylen-trideca-7,11-dienal enthält.

8. Verfahren nach einem der vorhergehenden Ansprüche, wobei das bei der Hydroformylierung (Schritt a) erhaltene Reaktionsgemisch wenigstens einem Aufarbeitungsschritt zur Abtrennung wenigstens einer der folgenden Komponenten:
- Hydroformylierungskatalysator,
- von den Verbindungen der Formel (I) verschiedenen Reaktionsprodukten,
- nicht umgesetzten Verbindungen der Formel (II),
- Lösungsmittel,
unterzogen wird.

9. Verfahren nach einem der Ansprüche 1 bis 8, wobei der eingesetzte Hydroformylierungskatalysator wenigstens eine Phosphorchelatverbindung der allgemeinen Formel (VI.1) 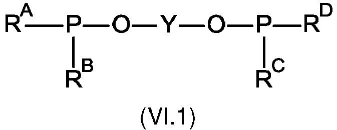 umfasst, worin
Y die zuvor angegebene Bedeutung besitzt,
die Reste R^{A}, R^{B}, R^{C} und R^{D} unabhängig voneinander ausgewählt sind unter Gruppen der Formeln VIII.a bis VIII.k 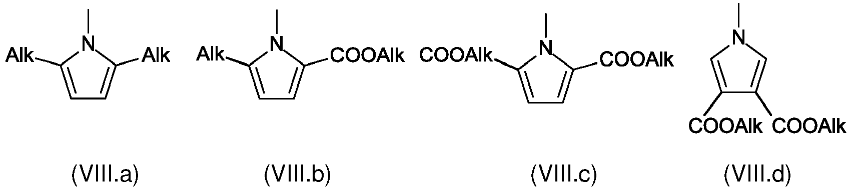 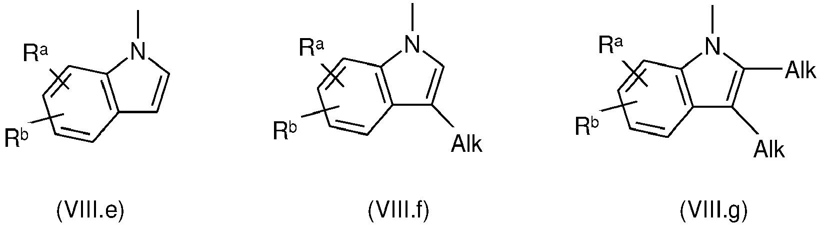 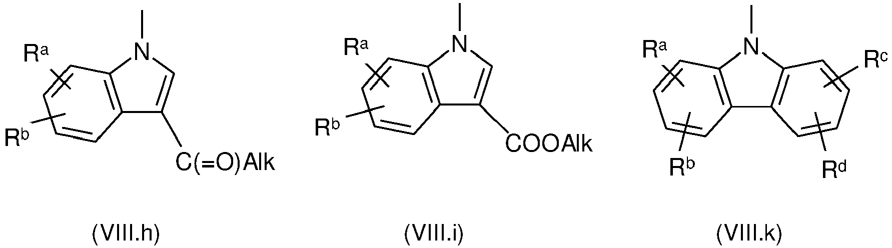 worin
Alk eine C₁-C₄-Alkylgruppe ist und
R^{a}, R^{b}, R^{c} und R^{d} unabhängig voneinander für Wasserstoff, C₁-C₄-Alkyl, C₁-C₄-Alkoxy, Formyl, Acyl, Halogen, C₁-C₄-Alkoxycarbonyl oder Carboxyl stehen.

10. Verfahren nach einem der Ansprüche 1 bis 8, wobei der eingesetzte Hydroformylierungskatalysator wenigstens eine Phosphorchelatverbindung der allgemeinen Formel (VI.2) 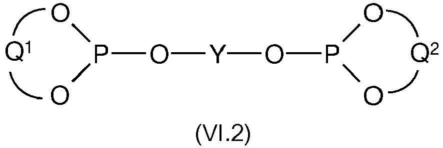 umfasst, worin
Y die zuvor angegebene Bedeutung besitzt,
Q¹ und Q² unabhängig voneinander für eine zweiwertige verbrückende Gruppe der allgemeinen Formel (IX) stehen, 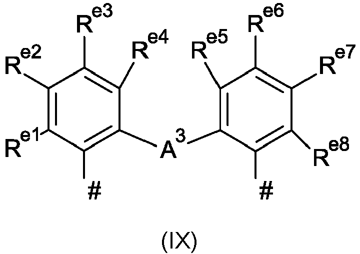
worin
# jeweils für eine Bindungsstelle an die Phosphit-Gruppe steht,
R^{e1}, R^{e2}, R^{e3}, R^{e4}, R^{e5}, Re6, R^{e7} und R^{e8} unabhängig voneinander für Wasserstoff, jeweils unsubstituiertes oder substituiertes Alkyl, Alkoxy, Cycloalkyl, Cycloalkoxy, Heterocycloalkyl, Heterocycloalkoxy, Aryl, Aryloxy, Hetaryl, Hetaryloxy,
Halogen, Hydroxy, Mercapto, Cyano, Nitro, Formyl, Acyl, Carboxy, Carboxylate, Alkylcarbonyloxy, Carbamoyl, Sulfo, Sulfonat oder NE¹²E¹³ stehen, worin E¹² und E¹³ jeweils gleiche oder verschiedene Reste, ausgewählt unter Wasserstoff, Alkyl, Cycloalkyl, Heterocycloalkyl, Aryl oder Hetaryl bedeuten,
wobei zwei benachbarte Reste R^{e1} bis R^{e8} gemeinsam mit den Kohlenstoffatomen des Benzolkerns, an die sie gebunden sind, auch für ein kondensiertes Ringsystem mit 1, 2 oder 3 weiteren Ringen stehen können,
A³ für eine Einfachbindung, O, S, NR^{a31}, SiR^{a32}R^{a33} oder C₁-C₄-Alkylen steht, das eine Doppelbindung und/oder einen Alkyl-, Cycloalkyl-, Heterocycloalkyl-, Aryl- oder Hetaryl-Substituenten aufweisen kann, oder durch O, S, NR^{a31} oder SiR^{a32}R^{a33} unterbrochen sein kann, wobei R^{a31}, R^{a32} und R^{a33} unabhängig voneinander für Wasserstoff, Alkyl, Cycloalkyl, Heterocycloalkyl, Aryl oder Hetaryl stehen.

11. Verfahren nach einem der vorhergehenden Ansprüche, wobei der eingesetzte Hydroformylierungskatalysator wenigstens eine Phosphorchelatverbindung als Liganden umfasst, die ausgewählt ist unter den Verbindungen:

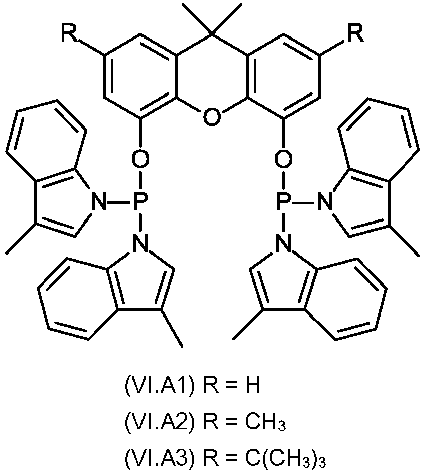

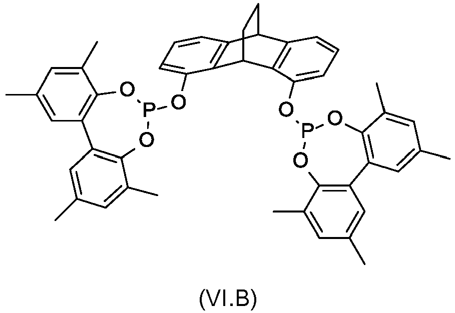

12. Verfahren zur Herstellung von (-)-Ambrox (V) 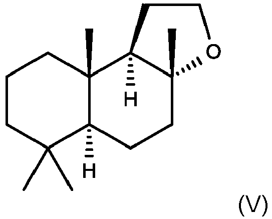 bei dem man
a1) (6*E*)-7,11-Dimethyl-3-methylidenedodeca-1,6,10-trien (II.1) 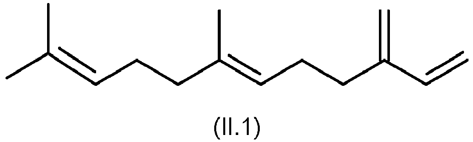 einer Umsetzung mit Kohlenmonoxid und Wasserstoff in Gegenwart eines Hydroformylierungskatalysators unterzieht, der einen Rhodiumkomplex mit wenigstens einer Phosphorchelatverbindung als Liganden umfasst, wobei ein Reaktionsgemisch erhalten wird, das wenigstens 50,1 Gew.-%, bezogen auf das Gesamtgewicht des Reaktionsgemischs, (7E)-8,12-Dimethyl-4-methylen-trideca-7,11-dienal (1.1) 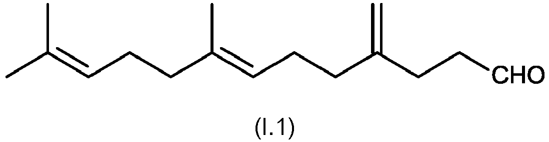 enthält,
b1) das in Schritt a) erhaltene Reaktionsgemisch oder eine an wenigstens einer Verbindung der allgemeinen Formel (I.1) angereicherte Fraktion daraus einer Hydrierung unterzieht, wobei ein Reaktionsgemisch erhalten wird, das die Verbindung (III.1) 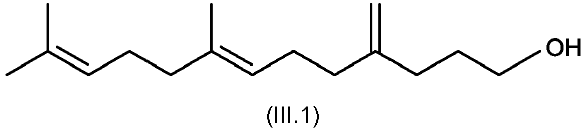 enthält,
c1) die Verbindung (III.1) einer zumindest teilweisen Isomerisierung unter Erhalt von (3*E,*7*E*)-Homofarnesol (IV.1) 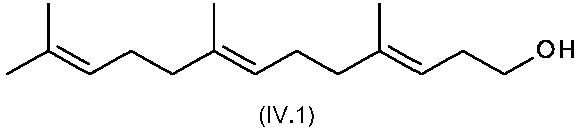 unterzieht,
d1) das (3*E,*7*E*)-Homofarnesol (IV.1) einer Cyclisierung unter Erhalt von (-)-Ambrox (V) unterzieht.

13. Verfahren nach Anspruch 12, wobei die Hydrierung in Schritt b1) in Gegenwart eines homogenen Rutheniumkatalysators erfolgt.

14. Verfahren nach einem der Ansprüche 12 oder 13, wobei die Isomerisierung in Schritt c1) in Gegenwart eines Katalysators der Formel
[Ru(dienyl)₂H]A
erfolgt, worin
dienyl für eine C₅-C₂₂-Kohlenwasserstoffgruppe steht, die eine Kohlenstoff-Kohlenstoff-Doppelbindung und eine Gruppe C=C-C- aufweist, und
A für ein schwach koordinierendes oder nicht koordinierendes Anion steht.

15. Verfahren nach einem der Ansprüche 12 bis 14, wobei die Cyclisierung in Schritt d1) in Gegenwart eines Polypeptids mit der Aktivität einer Homofarnesol-Ambrox-Cyclase als Enzym erfolgt.

## Claims

1. Process for the preparation of compounds of the general formula (I) and of descendant products thereof, 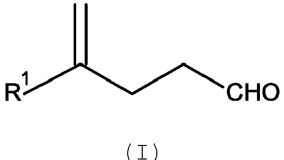 comprising at least one reaction step, in which at least one compound of the general formula (II) 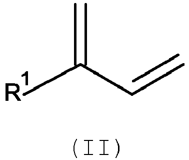 in which
R¹ is in each case linear or branched C₁-C₂₄-alkyl or C₂-C₂₄-alkenyl having 1, 2, 3 or more than 3 C-C double bonds,
is subjected to a hydroformylation by reaction with carbon monoxide and hydrogen in the presence of a hydroformylation catalyst which comprises a rhodium complex with at least one phosphorus chelate compound as ligand, wherein a reaction mixture is obtained which comprises at least 50.1% by weight, based on the total weight of the reaction mixture, of at least one compound of the general formula (I), wherein the hydroformylation catalyst used comprises at least one phosphorus chelate compound of the general formula (VI) 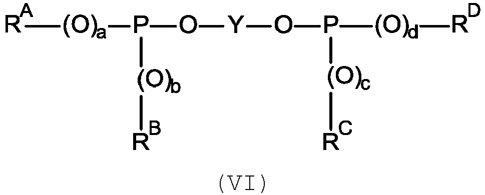 in which
R^{A}, R^{B}, R^{C} and R^{D}, independently of one another, are alkyl, cycloalkyl, heterocycloalkyl, aryl or hetaryl,
where the alkyl radicals R^{A}, R^{B}, R^{C} and R^{D} are unsubstituted or carry 1, 2, 3, 4 or 5 identical or different substituents which are selected from cycloalkyl, heterocycloalkyl, aryl, hetaryl, alkoxy, cycloalkoxy, heterocycloalkoxy, aryloxy, hetaryloxy, hydroxy, mercapto, polyalkylene oxide, polyalkyleneimine, COOH, carboxylate, SO₃H, sulfonate, NE¹E², NE¹E²E³⁺X⁻, halogen, nitro, formyl, acyl and cyano, in which E¹, E² and E³ are in each case identical or different radicals selected from hydrogen, alkyl, cycloalkyl or aryl, and X⁻ is an anion equivalent,
and where the cycloalkyl, heterocycloalkyl, aryl and hetaryl radicals R^{A}, R^{B}, R^{C} and R^{D} are in each case unsubstituted or carry 1, 2, 3, 4 or 5 identical or different substituents which are selected from alkyl and the substituents specified above for the alkyl radicals R^{A}, R^{B}, R^{C} and R^{D},
or
R^{A} and R^{B} and/or R^{C} and R^{D}, together with the phosphorus atom and, if present, the oxygen atoms to which they are bonded, are a 5- to 8-membered heterocycle which is optionally additionally annelated once, twice or three times with cycloalkyl, heterocycloalkyl, aryl or hetaryl, where the heterocycle and, if present, the annelated groups are, independently of one another, unsubstituted or carry one, two, three or four identical or different substituents which are selected from alkyl, cycloalkyl, heterocycloalkyl, aryl, hetaryl, hydroxy, mercapto, polyalkylene oxide, polyalkyleneimine, alkoxy, halogen, COOH, carboxylate, SO₃H,sulfonate, NE⁴E⁵, NE⁴E⁵E⁶⁺X⁻, nitro, alkoxycarbonyl, formyl, acyl and cyano, in which E⁴, E⁵ and E⁶ are in each case identical or different radicals, selected from hydrogen, alkyl, cycloalkyl and aryl, and X⁻ is an anion equivalent,
a, b, c and d, independently of one another, are 0 or 1, and
Y is selected from groups of the formula VII.a or VII.b 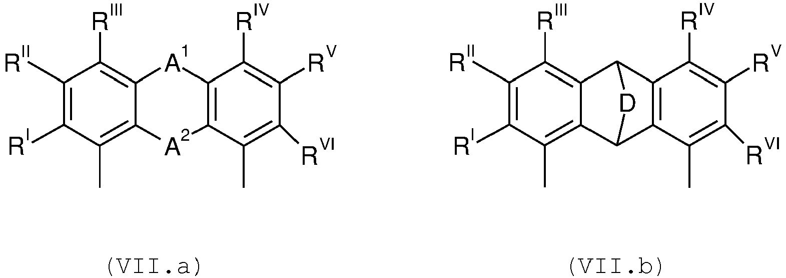
in which
R^{I}, R^{II}, R^{III}, R^{IV}, R^{V} and R^{VI}, independently of one another, are hydrogen, alkyl, cycloalkyl, heterocycloalkyl, aryl, hetaryl, hydroxy, mercapto, polyalkylene oxide, polyalkyleneimine, alkoxy, halogen, SO₃H, sulfonate, NE⁷E⁸, alkylene-NE⁷E⁸, trifluoromethyl, nitro, alkoxycarbonyl, carboxyl, formyl, acyl or cyano, in which E⁷ and E⁸ are in each case identical or different radicals selected from hydrogen, alkyl, cycloalkyl and aryl,
where two adjacent radicals R^{I} to R^{VI}, together with the carbon atoms of the benzene ring to which they are bonded, can also be a condensed ring system having 1, 2 or 3 further rings,
A¹ and A², independently of one another, are O, S, SiR^{E}R^{F}, NR^{G} or CR^{H}R^{K}, where R^{E}, R^{F}, R^{G}, R^{H} and R^{K}, independently of one another, are hydrogen, alkyl, cycloalkyl, heterocycloalkyl, aryl or hetaryl, and
D is a divalent bridging group of the general formula 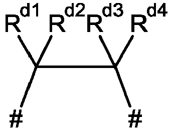
in which
# is in each case a binding site to the 9,10-dihydroanthracene backbone, and
R^{d1}, R^{d2}, R^{d3} and R^{d4}, independently of one another, are hydrogen, alkyl, cycloalkyl, aryl, halogen, trifluoromethyl, carboxyl, carboxylate or cyano,
where R^{d1} can also be, together with R^{d3}, the binding fraction of a double bond between the two carbon atoms to which R^{d1} and R^{d3} are bonded, and/or R^{d2} and R^{d4}, together with the carbon atoms to which they are bonded, can also be a 4- to 8-membered carbo- or heterocycle, which is optionally additionally annelated once, twice or three times with cycloalkyl, heterocycloalkyl, aryl or hetaryl, where the carbo- or heterocycle and, if present, the annelated groups are, independently of one another, unsubstituted or in each case carry one, two, three or four identical or different substituents which are selected from alkyl, cycloalkyl, heterocycloalkyl, aryl, hetaryl, COOR^{d5}, COC⁻M⁺, SO₃R^{d5}, SO₃⁻M⁺, NE⁹E¹⁰, alkylene-NE⁹E¹⁰, NE⁹E¹⁰E¹¹⁺ X⁻, alkylene-NE⁹E¹⁰E¹¹⁺ X⁻, OR^{d6}, SR^{d6}, (CHR^{f}CH₂O)_{y}R^{d6}, (CH₂N(E⁹))_{y}R^{d6}, (CH₂CH₂N (E⁹))_{y}R^{d6}, halogen, trifluoromethyl, nitro, formyl, acyl or cyano, in which
R^{d5}, E⁹, E¹⁰ and E¹¹ are in each case identical or different radicals selected from hydrogen, alkyl, cycloalkyl or aryl,
R^{d6} is hydrogen, methyl or ethyl,
M⁺ is a cation equivalent,
X⁻ is an anion equivalent, and
y is an integer from 1 to 120.

2. Process according to Claim 1, in which
a) at least one compound of the general formula (II) 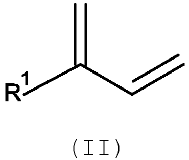 in which
R¹ is in each case linear or branched C₁-C₂₄-alkyl or C₂-C₂₄-alkenyl having 1, 2, 3 or more than 3 C-C double bonds,
is subjected to a reaction with carbon monoxide and hydrogen in the presence of a hydroformylation catalyst which comprises a rhodium complex with at least one phosphorus chelate compound as ligand, wherein a reaction mixture is obtained which comprises at least 50.1% by weight, based on the total weight of the reaction mixture, of at least one compound of the general formula (I),
b) the reaction mixture obtained in step a) or a fraction thereof enriched in at least one compound of the general formula (I) is subjected to a hydrogenation, wherein a reaction mixture is obtained which comprises at least one compound of the general formula (III)

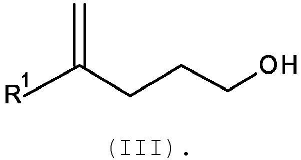

3. Process according to either of Claims 1 and 2, in which, additionally,
c) the at least one compound of the general formula (III) is subjected to an at least partial isomerization to give a compound of the general formula (IV)

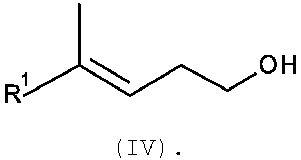

4. Process according to any one of the preceding claims, wherein the compounds of the formula (II) have only one vinyl group in the molecule.

5. Process according to any one of the preceding claims, wherein the compound of the general formula (II) is selected from isoprene, β-myrcene and β-farnesene.

6. Process according to any one of the preceding claims, wherein the reaction mixture obtained in the hydroformylation (step a)) comprises at least 55% by weight, preferably at least 60% by weight, in particular at least 65% by weight, based on the total weight of the reaction mixture, of at least one compound of the general formula (I).

7. Process according to any one of the preceding claims, comprising at least one reaction step in which β-farnesene is subjected to a reaction with carbon monoxide and hydrogen in the presence of a hydroformylation catalyst (= step a)), wherein a reaction mixture is obtained which comprises at least 50.1% by weight, based on the total weight of the reaction mixture, of (7E)-8,12-dimethyl-4-methylenetrideca-7,11-dienal.

8. Process according to any one of the preceding claims, wherein the reaction mixture obtained in the hydroformylation (step a)) is subjected to at least one work-up step to separate off at least one of the following components:
- hydroformylation catalyst,
- reaction products different from the compounds of the formula (I),
- unreacted compounds of the formula (II),
- solvents.

9. Process according to any one of Claims 1 to 8, wherein the hydroformylation catalyst used comprises at least one phosphorus chelate compound of the general formula (VI.1) 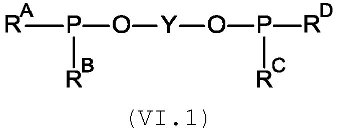 in which
Y has the meaning given above, and
the radicals R^{A}, R^{B}, R^{C} and R^{D}, independently of one another, are selected from groups of the formulae VIII.a to VIII.k 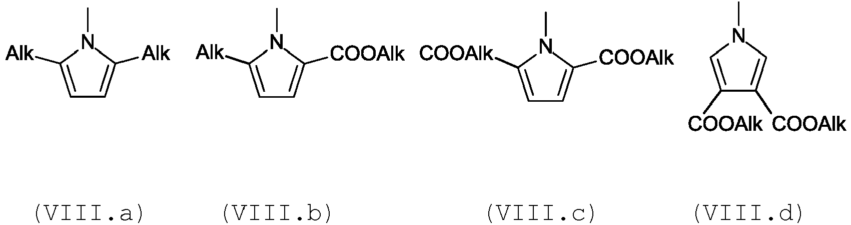 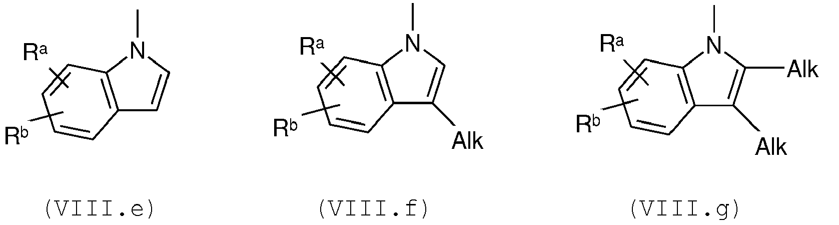 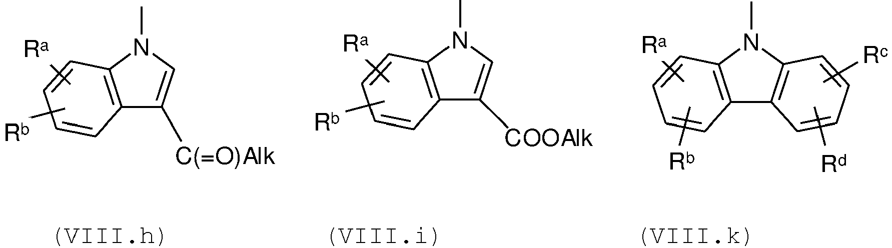 in which
Alk is a C₁-C₄-alkyl group and
R^{a}, R^{b}, R^{c}and R^{d}, independently of one another, are hydrogen, C₁-C₄-alkyl, C₁-C₄-alkoxy, formyl, acyl, halogen, C₁-C₄-alkoxycarbonyl or carboxyl.

10. Process according to any one of Claims 1 to 8, where the hydroformylation catalyst used comprises at least one phosphorus chelate compound of the general formula VI.2 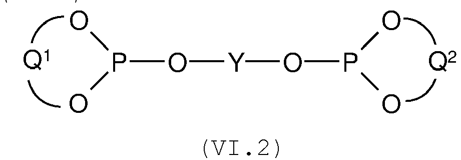 in which
Y has the meaning given above, and
Q¹ and Q², independently of one another, are a divalent bridging group of the general formula (IX), 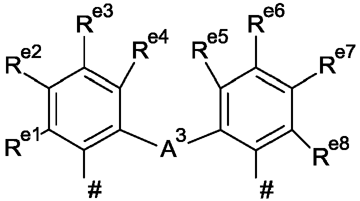 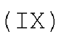
in which
# is in each case a binding site to the phosphite group,
R^{e1}, R^{e2}, R^{e3}, R^{e4}, R^{e5}, R^{e6}, R^{e7} and R^{e8}, independently of one another, are hydrogen, in each case unsubstituted or substituted alkyl, alkoxy, cycloalkyl, cycloalkoxy, heterocycloalkyl, heterocycloalkoxy, aryl, aryloxy, hetaryl, hetaryloxy, halogen, hydroxy, mercapto, cyano, nitro, formyl, acyl, carboxy, carboxylates, alkylcarbonyloxy, carbamoyl, sulfo, sulfonate or NE¹²E¹³, in which E¹² and E¹³ are in each case identical or different radicals selected from hydrogen, alkyl, cycloalkyl, heterocycloalkyl, aryl or hetaryl,
where two adjacent radicals R^{e1} to R^{e8}, together with the carbon atoms of the benzene ring to which they are bonded, can also be a condensed ring system having 1, 2 or 3 further rings, and
A³ is a single bond, O, S, NR^{a31}, SiR^{a32}R^{a33} or C₁-C₄-alkylene which can have a double bond and/or an alkyl, cycloalkyl, heterocycloalkyl, aryl or hetaryl substituent, or can be interrupted by O, S, NR^{a31} or SiR^{a32}R^{a33}, where R^{a31}, R^{a32} and R^{a33}, independently of one another, are hydrogen, alkyl, cycloalkyl, heterocycloalkyl, aryl or hetaryl.

11. Process according to any one of the preceding claims, wherein the hydroformylation catalyst used comprises at least one phosphorus chelate compound as ligand which is selected from the compounds:

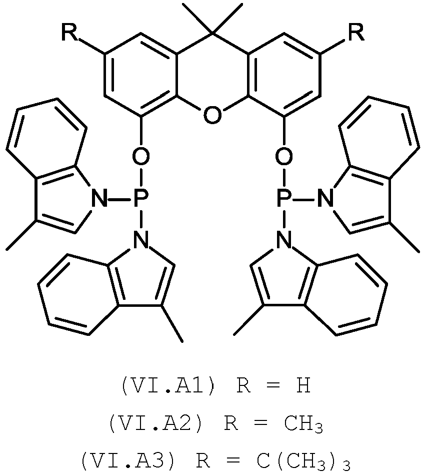

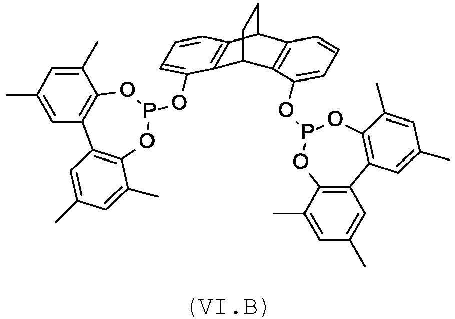

12. Process for the preparation of (-)-ambrox (V) 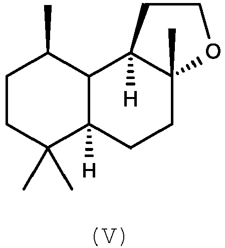 in which
a1) (6*E*)-7,11-dimethyl-3-methylidenedodeca-1,6,10-triene (II.1) 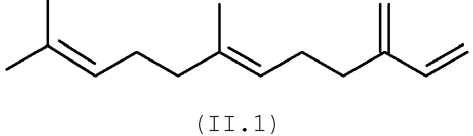 is subjected to a reaction with carbon monoxide and hydrogen in the presence of a hydroformylation catalyst which comprises a rhodium complex with at least one phosphorus chelate compound as ligand, wherein a reaction mixture is obtained which comprises at least 50.1% by weight, based on the total weight of the reaction mixture, of (7E)-8,12-dimethyl-4-methylenetrideca-7,11-dienal (I.1) 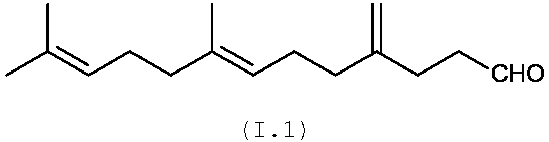
b1) the reaction mixture obtained in step a) or a fraction thereof enriched in at least one compound of the general formula (I.1) is subjected to a hydrogenation, wherein a reaction mixture is obtained which comprises the compound (III.1) 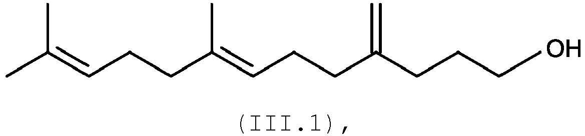
c1) the compound (III.1) is subjected to an at least partial isomerization, giving (3*E,*7*E*)-homofarnesol (IV.1) 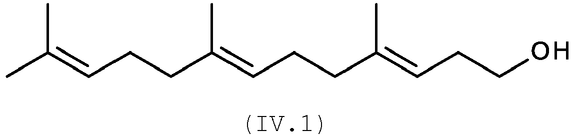
d1) the (3E,7E)-homofarnesol (IV.1) is subjected to a cyclization to give (-)-ambrox (V).

13. Process according to Claim 12, wherein the hydrogenation in step b1) takes place in the presence of a homogenous ruthenium catalyst.

14. Process according to either of Claims 12 and 13, wherein the isomerization in step c1) takes place in the presence of a catalyst of the formula
[Ru(dienyl)₂H]A
in which
dienyl is a C₅-C₂₂-hydrocarbon group which has a carbon-carbon double bond and a group C=C-C⁻, and
A is a weakly coordinating or noncoordinating anion.

15. Process according to any one of Claims 12 to 14, wherein the cyclization in step d1) takes place in the presence of a polypeptide with the activity of a homofarnesol-ambrox-cyclase as enzyme.

## Revendications

1. Procédé pour la préparation de composés de formule générale (I) et de produits dérivés de ceux-ci, 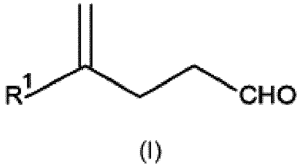 comprenant au moins une étape de réaction dans laquelle on soumet au moins un composé de formule générale (II) 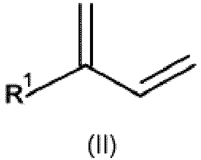 dans laquelle
R¹ représente à chaque fois C₁₋₂₄-alkyle ou C₂₋₂₄-alcényle linéaire ou ramifié comportant 1, 2, 3 ou plus de 3 doubles liaisons C-C,
à une hydroformylation par transformation avec du monoxyde de carbone et de l'hydrogène en présence d'un catalyseur d'hydroformylation, qui comprend un complexe du rhodium comportant au moins un composé chélatant au phosphore en tant que ligands, un mélange réactionnel étant obtenu qui contient au moins 50,1 % en poids, par rapport au poids total du mélange réactionnel, d'au moins un composé de formule générale (I), le catalyseur d'hydroformylation comprenant au moins un composé chélatant au phosphore de formule générale (VI) 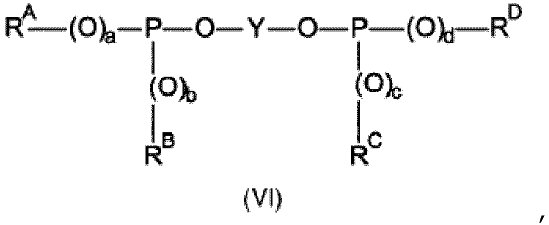 dans laquelle
R^{A}, R^{B}, R^{C} et R^{D} représentent indépendamment les uns des autres alkyle, cycloalkyle, hétérocycloalkyle, aryle ou hétaryle,
les radicaux alkyle R^{A}, R^{B}, R^{C} et R^{D} étant non substitués ou portant 1, 2, 3, 4 ou 5 substituants identiques ou différents, qui sont choisis parmi cycloalkyle, hétérocycloalkyle, aryle, hétaryle, alcoxy, cycloalcoxy, hétérocycloalcoxy, aryloxy, hétaryloxy, hydroxy, mercapto, poly(oxyde d'alkylène), polyalkylèneimine, COOH, carboxylate, SO₃H, sulfonate, NE¹E², NE¹E²E³⁺X⁻, halogène, nitro, formyle, acyle et cyano, dans lesquels E¹, E² et E³ signifient à chaque fois des radicaux identiques ou différents choisis parmi hydrogène, alkyle, cycloalkyle et aryle et X⁻ représente un équivalent anion,
et les radicaux cycloalkyle, hétérocycloalkyle, aryle et hétaryle R^{A}, R^{B}, R^{C} et R^{D} étant à chaque fois non substitués ou portant 1, 2, 3, 4 ou 5 substituants identiques ou différents, qui sont choisis parmi alkyle et les substituants mentionnés précédemment pour les radicaux alkyle R^{A}, R^{B}, R^{C} et R^{D},
ou
R^{A} et R^{B} et/ou R^{C} et R^{D}, conjointement avec l'atome de phosphore et, s'ils sont présents, les atomes d'oxygène, auxquels ils sont liés, représentent un hétérocycle de 5 à 8 chaînons, qui peut être éventuellement de plus annelé une fois, deux fois ou trois fois avec cycloalkyle, hétérocycloalkyle, aryle ou hétaryle, l'hétérocycle et, s'ils sont présents, les groupes annelés étant indépendamment les uns des autres non substitués ou portant un, deux, trois ou quatre substituants identiques ou différents, qui sont choisis parmi alkyle, cycloalkyle, hétérocycloalkyle, aryle, hétaryle, hydroxy, mercapto, poly(oxyde d'alkylène), polyalkylèneimine, alcoxy, halogène, COOH, carboxylate, SO₃H,sulfonate, NE⁴E⁵, NE⁴E⁵E⁶⁺X⁻, nitro, alcoxycarbonyle, formyle, acyle et cyano, dans lesquels E⁴, E⁵ et E⁶ signifient à chaque fois des radicaux identiques ou différents choisis parmi hydrogène, alkyle, cycloalkyle, et aryle et X⁻ représente un équivalent anion,
a, b, c et d représentent indépendamment les uns des autres 0 ou 1,
Y est choisi parmi les groupes des formules VII.a et VII.b 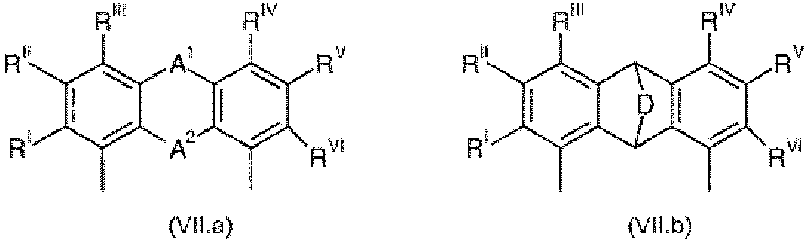
dans lesquelles
R^{I}, R^{II}, R^{III}, R^{IV}, R^{V} et R^{VI} représentent indépendamment les uns des autres hydrogène, alkyle, cycloalkyle, hétérocycloalkyle, aryle, hétaryle, hydroxy, mercapto, poly(oxyde d'alkylène), polyalkylèneimine, alcoxy, halogène, SO₃H, sulfonate, NE⁷E⁸, alkylène-NE⁷E⁸, trifluorométhyle, nitro, alcoxycarbonyle, carboxyle, formyle, acyle ou cyano, dans lesquels E⁷ et E⁸ signifient à chaque fois des radicaux identiques ou différents choisis parmi hydrogène, alkyle, cycloalkyle et aryle,
deux radicaux voisins R^{I} à R^{VI}, conjointement avec les atomes de carbone du noyau benzénique auxquels ils sont liés, pouvant également représenter un système cyclique condensé comportant 1, 2 ou 3 cycles supplémentaires,
A¹ et A² représentent indépendamment l'un de l'autre O, S, SiR^{E}R^{F}, NR^{G} ou CR^{H}R^{K},
R^{E}, R^{F}, R^{G}, R^{H} et R^{K} représentant indépendamment les uns des autres hydrogène, alkyle, cycloalkyle, hétérocycloalkyle, aryle ou hétaryle,
D représente un groupe pontant divalent de formule générale 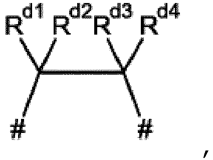
dans laquelle
# représente à chaque fois un site de liaison au squelette 9,10-dihydroanthracène,
R^{d1}, R^{d2}, R^{d3} et R^{d4} représentent indépendamment les uns des autres hydrogène, alkyle, cycloalkyle, aryle, halogène, trifluorométhyle, carboxyle, carboxylate ou cyano,
R^{d1} pouvant représenter également, conjointement avec R^{d3}, la fraction de liaison d'une double liaison entre les deux atomes de carbone auxquels R^{d1} et R^{d3} sont liés, et/ou R^{d2} et R^{d4}, conjointement avec les atomes de carbone auxquels ils sont liés, pouvant également représenter un carbocycle ou un hétérocycle de 4 à 8 chaînons, qui est éventuellement de plus annelé une fois, deux fois ou trois fois avec cycloalkyle, hétérocycloalkyle, aryle ou hétaryle, le carbocycle ou l'hétérocycle et, s'ils sont présents, les groupes annelés étant indépendamment les uns des autres non substitués ou portant chacun un, deux, trois ou quatre substituants identiques ou différents qui sont choisis parmi alkyle, cycloalkyle, hétérocycloalkyle, aryle, hétaryle, COOR^{d5}, COO⁻ M⁺, SO₃R^{d5}, SO₃⁻M⁺, NE⁹E¹⁰, alkylène-NE⁹E¹⁰, NE⁹E¹⁰E¹¹⁺ X⁻, alkylène-NE⁹E¹⁰E¹¹⁺ X⁻, OR^{d6}, SR^{d6}, (CHR^{f}CH₂O)_{y}R^{d6}, (CH₂N(E⁹))_{y}R^{d6}, (CH₂CH₂N(E⁹))_{y}R^{d6}, halogène, trifluorométhyle, nitro, formyle, acyle ou cyano, dans lesquels
R^{d5}, E⁹, E¹⁰ et E¹¹ signifient des radicaux à chaque fois identiques ou différents choisis parmi hydrogène, alkyle, cycloalkyle et aryle,
R^{d6} représente hydrogène, méthyle ou éthyle,
M⁺ représente un équivalent cation,
X⁻ représente un équivalent anion, et
y représente un nombre entier de 1 à 120.

2. Procédé selon la revendication 1, dans lequel
a) on soumet au moins un composé de formule générale (II) 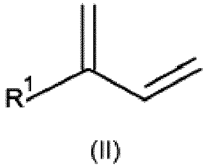 dans laquelle
R¹ représente à chaque fois C₁₋₂₄-alkyle ou C₂₋₂₄-alcényle linéaire ou ramifié comportant 1, 2, 3 ou plus de 3 doubles liaisons C-C,
à une transformation avec du monoxyde de carbone et de l'hydrogène en présence d'un catalyseur d'hydroformylation, qui comprend un complexe du rhodium comportant au moins un composé chélatant au phosphore en tant que ligands, un mélange réactionnel étant obtenu qui contient au moins 50,1 % en poids, par rapport au poids total du mélange réactionnel, d'au moins un composé de formule générale (I),
b) on soumet le mélange réactionnel obtenu dans l'étape a), ou une fraction de celui-ci enrichie en au moins un composé de formule générale (I), à une hydrogénation, un mélange réactionnel étant obtenu qui contient au moins un composé de formule générale (III)

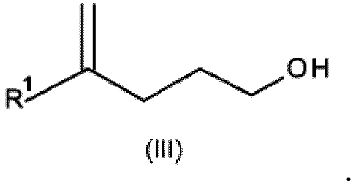

3. Procédé selon l'une quelconque des revendications 1 et 2, dans lequel de plus
c) on soumet l'au moins un composé de formule générale (III) à une isomérisation au moins partielle avec obtention d'un composé de formule générale (IV)

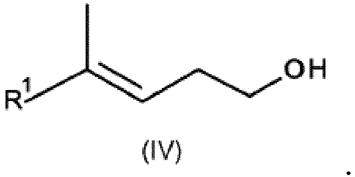

4. Procédé selon l'une quelconque des revendications précédentes, les composés de formule (II) ne présentant qu'un seul groupe vinyle dans la molécule.

5. Procédé selon l'une quelconque des revendications précédentes, le composé de formule générale (II) étant choisi parmi l'isoprène, le β-myrcène et le β-farnésène.

6. Procédé selon l'une quelconque des revendications précédentes, le mélange réactionnel obtenu dans l'hydroformylation (étape a)) contenant au moins 55 % en poids, préférablement au moins 60 % en poids, en particulier au moins 65 % en poids, par rapport au poids total du mélange réactionnel, d'au moins un composé de formule générale (I).

7. Procédé selon l'une quelconque des revendications précédentes, comprenant au moins une étape de réaction dans laquelle on soumet du β-farnésène à une transformation avec du monoxyde de carbone et de l'hydrogène en présence d'un catalyseur d'hydroformylation (= étape a)), un mélange réactionnel étant obtenu qui contient au moins 50,1 % en poids, par rapport au poids total du mélange réactionnel, de (7E)-8,12-diméthyl-4-méthylènetridéca-7,11-diénal.

8. Procédé selon l'une quelconque des revendications précédentes, le mélange réactionnel obtenu dans l'hydroformylation (étape a)) étant soumis à au moins une étape de traitement de séparation d'au moins l'un des composants suivants :
- catalyseur d'hydroformylation,
- produits de réaction différents des composés de formule (I),
- composés de formule (II) n'ayant pas réagi,
- solvants.

9. Procédé selon l'une quelconque des revendications 1 à 8, le catalyseur d'hydroformylation utilisé comprenant au moins un composé chélatant au phosphore de formule générale (VI.1) 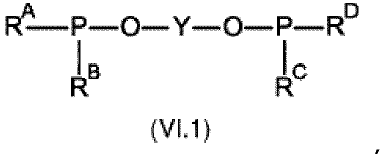 dans laquelle
Y possède la signification mentionnée précédemment,
les radicaux R^{A}, R^{B}, R^{C} et R^{D} sont choisis indépendamment les uns des autres parmi les groupes de formules VIII.a à VIII.k 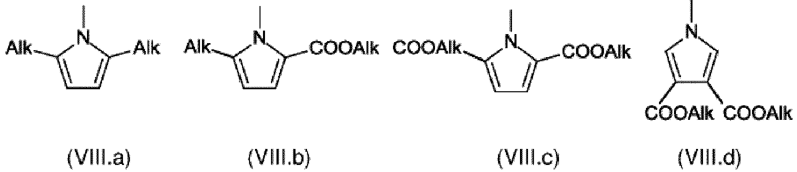 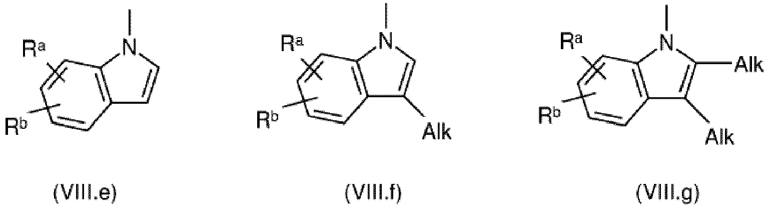 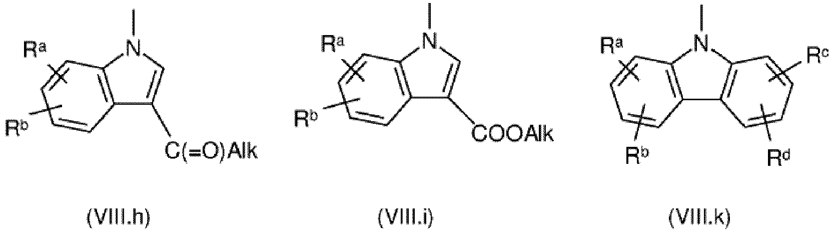 dans lesquelles
Alk est un groupe C₁₋₄-alkyle et
R^{a}, R^{b}, R^{c} et R^{d} représentent indépendamment les uns des autres hydrogène, C₁₋₄-alkyle, C₁₋₄-alcoxy, formyle, acyle, halogène, C₁₋₄-alcoxycarbonyle ou carboxyle.

10. Procédé selon l'une quelconque des revendications 1 à 8, le catalyseur d'hydroformylation utilisé comprenant au moins un composé chélatant au phosphore de formule générale (VI.2) 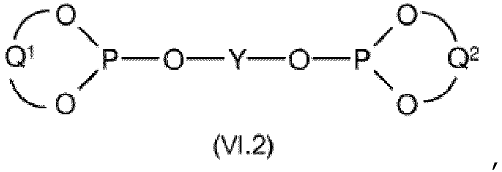 dans laquelle
Y possède la signification mentionnée précédemment,
Q¹ et Q² représentent indépendamment l'un de l'autre un groupe pontant divalent de formule générale (IX), 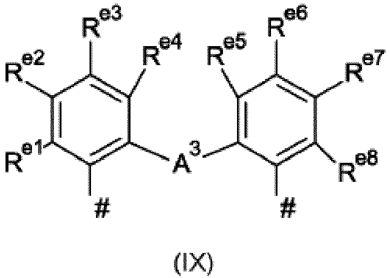
dans laquelle
# représente à chaque fois un site de liaison au groupe phosphite,
R^{e1}, R^{e2}, R^{e3}, R^{e4}, R^{e5}, R^{e6}, R^{e7} et R^{e8} représentent indépendamment les uns des autres hydrogène, alkyle, alcoxy, cycloalkyle, cycloalcoxy, hétérocycloalkyle, hétérocycloalcoxy, aryle, aryloxy, hétaryle, hétaryloxy, à chaque fois non substitué ou substitué,
halogène, hydroxy, mercapto, cyano, nitro, formyle, acyle, carboxy, carboxylate, alkylcarbonyloxy, carbamoyle, sulfo, sulfonate ou NE¹²E¹³, dans lequel E¹² et E¹³ signifient des radicaux à chaque fois identiques ou différents, choisis parmi hydrogène, alkyle, cycloalkyle, hétérocycloalkyle, aryle et hétaryle,
deux radicaux voisins R^{e1} à R^{e8}, conjointement avec les atomes de carbone du noyau benzénique auxquels ils sont liés, pouvant également représenter un système cyclique condensé comportant 1, 2 ou 3 cycles supplémentaires,
A³ représente une simple liaison, O, S, NR^{a31}, SiR^{a32}R^{a33} ou C₁-C₄-alkylène, qui peut présenter une double liaison et/ou un substituant alkyle, cycloalkyle, hétérocycloalkyle, aryle ou hétaryle, ou peut être interrompu par O, S, NR^{a31} ou SiR^{a32}R^{a33} ; R^{a31}, R^{a32} et R^{a33} représentant indépendamment les uns des autres hydrogène, alkyle, cycloalkyle, hétérocycloalkyle, aryle ou hétaryle.

11. Procédé selon l'une quelconque des revendications précédentes, le catalyseur d'hydroformylation utilisé comprenant au moins un composé chélatant au phosphore en tant que ligands, qui est choisi parmi les composés :

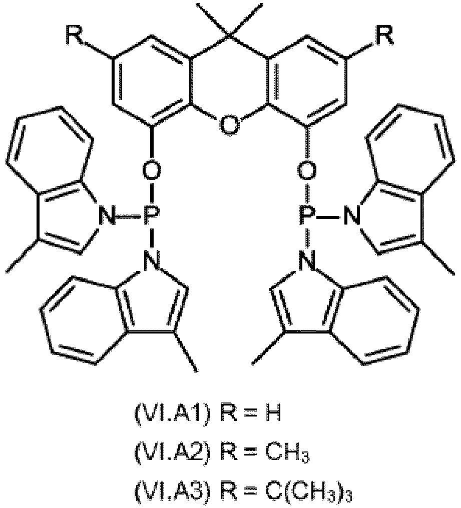

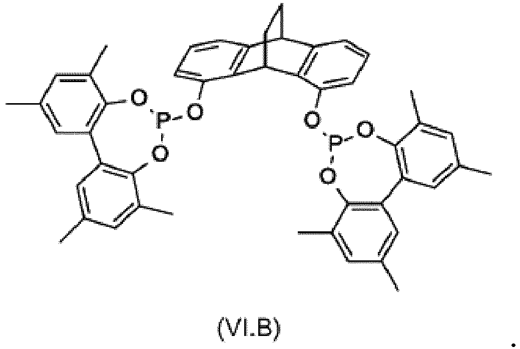

12. Procédé pour la préparation de (-)-ambrox (V) 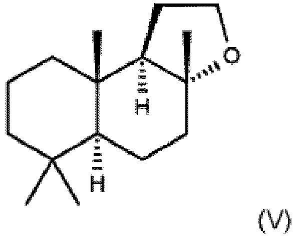 dans lequel
a1) on soumet du (6*E*)-7,11-diméthyl-3-méthylidènedodéca-1,6,10-triène (II.1) 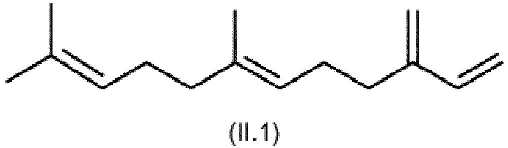 à une transformation avec du monoxyde de carbone et de l'hydrogène en présence d'un catalyseur d'hydroformylation, qui comprend un complexe du rhodium comportant au moins un composé chélatant au phosphore en tant que ligands, un mélange réactionnel étant obtenu qui contient au moins 50,1 % en poids, par rapport au poids total du mélange réactionnel, de (7E)-8,12-diméthyl-4-méthylènetridéca-7,11-diénal (I.1) 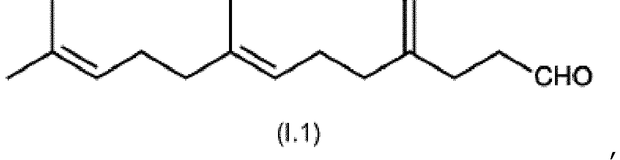
b1) on soumet le mélange réactionnel obtenu dans l'étape a), ou une fraction de celui-ci enrichie en au moins un composé de formule générale (I.1), à une hydrogénation, un mélange réactionnel étant obtenu qui contient le composé (III.1) 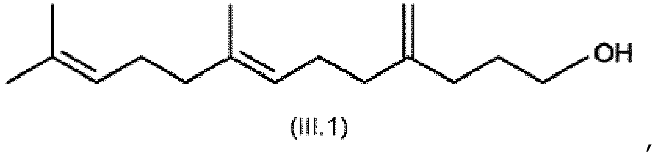
c1) on soumet le composé (III.1) à une isomérisation au moins partielle avec obtention de (3*E*,7*E*)-homofarnésol (IV.1) 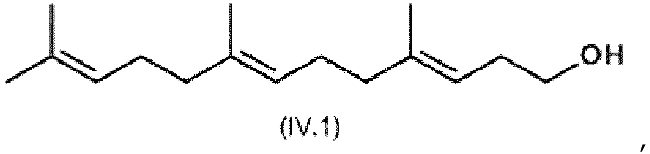
d1) on soumet le (3*E*,7*E*)-homofarnésol (IV.1) à une cyclisation avec obtention de (-)-ambrox (V) .

13. Procédé selon la revendication 12, l'hydrogénation dans l'étape b1) étant réalisée en présence d'un catalyseur homogène au ruthénium.

14. Procédé selon l'une quelconque des revendications 12 et 13, l'isomérisation dans l'étape c1) étant réalisée en présence d'un catalyseur de formule
[Ru(diényl)₂H]A
dans laquelle
diényl représente un groupe hydrocarboné en C_{5- * 22}, qui présente une double liaison carbone-carbone et un groupe C=C-C⁻, et
A représente un anion faiblement coordinant ou non coordinant.

15. Procédé selon l'une quelconque des revendications 12 à 14, la cyclisation dans l'étape d1) étant réalisée en présence d'un polypeptide comportant l'activité d'une homofarnésol-ambrox-cyclase en tant qu'enzyme.
